# EUROPEAN PATENT SPECIFICATION

(11) **EP 2 119 770 B1**
(45) Date of publication and mention of the grant of the patent: **23.10.2013**
(21) Application number: 08156108.6
(22) Date of filing: 13.05.2008
(51) Int. Cl.: C12P 7/24, C12P 41/00, C12N 9/02

(54) **Production of alpha-oxyfunctionalized carbonyl compounds**
Erzeugung von Alpha-oxyfunktionalisierten Carbonyl-Zusammensetzungen
Production de composés carbonyle alpha-oxyfonctionnalisés

(43) Date of publication of application: 18.11.2009
(73) Proprietor: Rheinische Friedrich-Wilhelms-Universität Bonn, 53113 Bonn (DE)
(72) Inventor: Deppenmeier, Uwe, Prof. Dr., 53797 Lohmar (DE)
(74) Representative: von Kreisler Selting Werner

(56) References cited:
- EP-A- 0 976 827
- PRUST CHRISTINA ET AL: "Complete genome sequence of the acetic acid bacterium Gluconobacter oxydans" NATURE BIOTECHNOLOGY, NATURE PUBLISHING GROUP, NEW YORK, NY, US, vol. 23, no. 2, 23 January 2005 (2005-01-23), pages 195-200, XP002377638 ISSN: 1087-0156 -& DATABASE UniProt [Online] 1 March 2005 (2005-03-01), "SubName: Full=Putative oxidoreductase; EC=<A HREF="http://srs.ebi.ac.uk/srsbin/cgi-bin/ wgetz?[enzyme-ECNumber:1.1.1.*]+-e">1.1.1. -</A>;" XP002497717 retrieved from EBI accession no. UNIPROT:Q5FQJ0 Database accession no. Q5FQJ0 -& "Putative 2,5-diketo-D-gluconic acid reductase (EC <A HREF=http://srs.ebi.ac.uk/srsbin/cgi-bin/w getz?[enzyme-ECNumber:1.1.1 .*]+-e>1.1.1.-</A>)" UNIPROT,, [Online] 1 January 2005 (2005-01-01), XP002487780 Retrieved from the Internet: URL:http://srs.ebi.ac.uk/srsbin/cgi-bin/wg etz?[enzyme-ECNumber:1.1.1.*]+- e>1.1.1.-</A>)> [retrieved on 2005-03-01]
- CHEN CHING-NEN ET AL: "Associating protein activities with their genes: rapid identification of a gene encoding a methylglyoxal reductase in the yeast Saccharomyces cerevisiae." YEAST (CHICHESTER, ENGLAND) 30 APR 2003, vol. 20, no. 6, 30 April 2003 (2003-04-30), pages 545-554, XP002487486 ISSN: 0749-503X
- JOHANSON T ET AL: "Strain engineering for stereoselective bioreduction of dicarbonyl compounds by yeast reductases" FEMS YEAST RESEARCH, ELSEVIER SCIENCE, TOKYO, NL, vol. 5, no. 6-7, 1 April 2005 (2005-04-01), pages 513-525, XP004789302 ISSN: 1567-1356
- SCHWEIGER PAUL ET AL: "Overproduction and characterization of two distinct aldehyde-oxidizing enzymes from Gluconobacter oxydans 621H" JOURNAL OF MOLECULAR MICROBIOLOGY AND BIOTECHNOLOGY, vol. 13, no. 1-3, 2007, pages 147-155, XP009102850 ISSN: 1464-1801
- NAKAGAWA JUNICHI ET AL: "Molecular characterization of mammalian dicarbonyl/L-xylulose reductase and its localization in kidney" JOURNAL OF BIOLOGICAL CHEMISTRY, vol. 277, no. 20, 17 May 2002 (2002-05-17), pages 17883-17891, XP002497716 ISSN: 0021-9258

### SEQUENCE LISTING

<110> Rheinische Friedrich-Wilhelms-Universitaet Bonn
<120> Production of Alpha-Oxyfunctionalized Carbonyl Compounds
<130> 072983ep
<160> 33
<170> PatentIn version 3.3
<210> 1
   <211> 1032
   <212> DNA
   <213> Gluconobacter oxydans
<220>
   <221> CDS
   <222> (1)..(1029)
<400> 1
   atg gct gat aca atg ctc gcc gcc gtc gtc cgt gaa ttc ggc aag ccg        48
   Met Ala Asp Thr Met Leu Ala Ala Val Val Arg Glu Phe Gly Lys Pro
   1               5                   10                  15
   ctc tcc atc gag cgg cta ccc atc ccg gac atc aag ccc cac cag atc        96
   Leu Ser Ile Glu Arg Leu Pro Ile Pro Asp Ile Lys Pro His Gln Ile
               20                  25                  30
   ctc gtg aag gtc gat acc tgt ggc gtc tgc cac act gac ctg cac gcc       144
   Leu Val Lys Val Asp Thr Cys Gly Val Cys His Thr Asp Leu His Ala
           35                  40                  45
   gcg cgc ggg gac tgg ccg tcc aag ccc aac ccg ccg ttc att ccc ggg       192
   Ala Arg Gly Asp Trp Pro Ser Lys Pro Asn Pro Pro Phe Ile Pro Gly
       50                  55                  60
   cat gaa ggc gtc gga cac atc gtc gcc gtc ggc agt cag gtc ggc gat       240
   His Glu Gly Val Gly His Ile Val Ala Val Gly Ser Gln Val Gly Asp
   65                  70                  75                      80
   ttc gtc aag acc ggc gat gtc gtg ggc gtg ccc tgg ctc tac tcc gcc       288
   Phe Val Lys Thr Gly Asp Val Val Gly Val Pro Trp Leu Tyr Ser Ala
                   85                  90                  95
   tgc ggt cac tgc gaa cac tgt ctg ggc ggc tgg gaa aca ctc tgc gaa       336
   Cys Gly His Cys Glu His Cys Leu Gly Gly Trp Glu Thr Leu Cys Glu
               100                 105                 110
   aag cag gac gac acc ggc tac acc gtc aat ggc tgc ttc gcc gaa tat       384
   Lys Gln Asp Asp Thr Gly Tyr Thr Val Asn Gly Cys Phe Ala Glu Tyr
           115                 120                 125
   gtc gtg gca gac ccg aac tac gtc gca cac ctg ccc tcg acc atc gac       432
   Val Val Ala Asp Pro Asn Tyr Val Ala His Leu Pro Ser Thr Ile Asp
       130                 135                 140
   ccg ctt cag gcc tcg ccg gtc ctg tgc gcg ggg ctg acg gtc tat aag       480
   Pro Leu Gln Ala Ser Pro Val Leu Cys Ala Gly Leu Thr Val Tyr Lys
   145                 150                 155                 160
   ggc ctg aaa atg acg gag gcc cgc ccc ggc cag tgg gtc gca gtc tcg        528
   Gly Leu Lys Met Thr Glu Ala Arg Pro Gly Gln Trp Val Ala Val Ser
                   165                 170                 175
   ggc gtc ggc ggt ctc ggc cag atg gcc gtg cag tac gcc gtc gcc atg        576
   Gly Val Gly Gly Leu Gly Gln Met Ala Val Gln Tyr Ala Val Ala Met
               180                 185                 190
   ggc atg aat gtc gtc gcg gtg gac atc gat gac gaa aaa ctc gcc aca        624
   Gly Met Asn Val Val Ala Val Asp Ile Asp Asp Glu Lys Leu Ala Thr
           195                 200                 205
   gcc aaa aag ctc ggc gca tcc ctg acc gtc aac gcc aag gac acg gac        672
   Ala Lys Lys Leu Gly Ala Ser Leu Thr Val Asn Ala Lys Asp Thr Asp
       210                 215                 220
   ccg gcc agg ttc atc cag cag cag atc ggc ggc gca cat ggc gct ctc        720
   Pro Ala Arg Phe Ile Gln Gln Gln Ile Gly Gly Ala His Gly Ala Leu
   225                 230                 235                 240
   gtc acc gct gtc gga cgg acg gcg ttt tcg cag gcc atg ggc tat gcc        768
   Val Thr Ala Val Gly Arg Thr Ala Phe Ser Gln Ala Met Gly Tyr Ala
                   245                 250                 255
   cgc cgc ggc ggc acc atc gtc ctg aac gga ctg ccg ccc ggc gat ttc        816
   Arg Arg Gly Gly Thr Ile Val Leu Asn Gly Leu Pro Pro Gly Asp Phe
               260                 265                 270
   ccg gtc tcg atc ttc gac atg gtc atg aac ggc acc acc atc cgt ggc        864
   Pro Val Ser Ile Phe Asp Met Val Met Asn Gly Thr Thr Ile Arg Gly
           275                 280                 285
   tcc atc gtc gga aca cgg ctg gac atg atc gag gcc atg gat ttc ttc        912
   Ser Ile Val Gly Thr Arg Leu Asp Met Ile Glu Ala Met Asp Phe Phe
       290                 295                 300
   gcc cgc ggc aag gtc aaa tcc gtc gtc acc ccc gga aaa ctt gaa aac        960
   Ala Arg Gly Lys Val Lys Ser Val Val Thr Pro Gly Lys Leu Glu Asn
   305                 310                 315                 320
   atc aat acg atc ttc gac gat ctg cag aat ggt cgc ctc gaa ggc cgg       1008
   Ile Asn Thr Ile Phe Asp Asp Leu Gln Asn Gly Arg Leu Glu Gly Arg
                   325                 330                 335
   aca gtg ctc gac ttc cgg tcc tga                                       1032
   Thr Val Leu Asp Phe Arg Ser
               340
<210> 2
   <211> 343
   <212> PRT
   <213> Gluconobacter oxydans
<400> 2
   Met Ala Asp Thr Met Leu Ala Ala Val Val Arg Glu Phe Gly Lys Pro
   1               5                   10                  15
   Leu Ser Ile Glu Arg Leu Pro Ile Pro Asp Ile Lys Pro His Gln Ile
               20                  25                  30
   Leu Val Lys Val Asp Thr Cys Gly Val Cys His Thr Asp Leu His Ala
           35                  40                  45
   Ala Arg Gly Asp Trp Pro Ser Lys Pro Asn Pro Pro Phe Ile Pro Gly
       50                  55                  60
   His Glu Gly Val Gly His Ile Val Ala Val Gly Ser Gln Val Gly Asp
   65                  70                  75                  80
   Phe Val Lys Thr Gly Asp Val Val Gly Val Pro Trp Leu Tyr Ser Ala
                   85                  90                  95
   Cys Gly His Cys Glu His Cys Leu Gly Gly Trp Glu Thr Leu Cys Glu
               100                 105                 110
   Lys Gln Asp Asp Thr Gly Tyr Thr Val Asn Gly Cys Phe Ala Glu Tyr
           115                 120                 125
   Val Val Ala Asp Pro Asn Tyr Val Ala His Leu Pro Ser Thr Ile Asp
       130                 135                 140
   Pro Leu Gln Ala Ser Pro Val Leu Cys Ala Gly Leu Thr Val Tyr Lys
   145                 150                 155                 160
   Gly Leu Lys Met Thr Glu Ala Arg Pro Gly Gln Trp Val Ala Val Ser
                   165                 170                 175
   Gly Val Gly Gly Leu Gly Gln Met Ala Val Gln Tyr Ala Val Ala Met
               180                 185                 190
   Gly Met Asn Val Val Ala Val Asp Ile Asp Asp Glu Lys Leu Ala Thr
           195                 200                 205
   Ala Lys Lys Leu Gly Ala Ser Leu Thr Val Asn Ala Lys Asp Thr Asp
       210                 215                 220
   Pro Ala Arg Phe Ile Gln Gln Gln Ile Gly Gly Ala His Gly Ala Leu
   225                 230                 235                 240
   Val Thr Ala Val Gly Arg Thr Ala Phe Ser Gln Ala Met Gly Tyr Ala
                   245                 250                 255
   Arg Arg Gly Gly Thr Ile Val Leu Asn Gly Leu Pro Pro Gly Asp Phe
               260                 265                 270
   Pro Val Ser Ile Phe Asp Met Val Met Asn Gly Thr Thr Ile Arg Gly
           275                 280                 285
   Ser Ile Val Gly Thr Arg Leu Asp Met Ile Glu Ala Met Asp Phe Phe
       290                 295                 300
   Ala Arg Gly Lys Val Lys Ser Val Val Thr Pro Gly Lys Leu Glu Asn
   305                 310                 315                 320
   Ile Asn Thr Ile Phe Asp Asp Leu Gln Asn Gly Arg Leu Glu Gly Arg
                   325                 330                 335
   Thr Val Leu Asp Phe Arg Ser
               340
<210> 3
   <211> 1086
   <212> DNA
   <213> Gluconobacter oxydans
<220>
   <221> CDS
   <222> (1)..(1083)
<400> 3
   atg cca acc ctg ttc gat ccc att gat ttc ggt ccc att cac gcg aaa        48
   Met Pro Thr Leu Phe Asp Pro Ile Asp Phe Gly Pro Ile His Ala Lys
   1               5                   10                  15
   aac cgg atc gtg atg tcc ccg ctg acg cgc gga cgt gct gac aag gag        96
   Asn Arg Ile Val Met Ser Pro Leu Thr Arg Gly Arg Ala Asp Lys Glu
               20                  25                  30
   gcc gtt ccg acc ccc atc atg gcg gaa tac tac gcc cag cgc gcc agt       144
   Ala Val Pro Thr Pro Ile Met Ala Glu Tyr Tyr Ala Gln Arg Ala Ser
           35                  40                  45
   gcc ggg ctg atc atc acg gaa gcc acg ggt atc tcc cgc gaa ggt ctg       192
   Ala Gly Leu Ile Ile Thr Glu Ala Thr Gly Ile Ser Arg Glu Gly Leu
       50                  55                  60
   ggc tgg ccg ttc gca ccg gga atc tgg tcc gat gcg cag gtc gaa gcc       240
   Gly Trp Pro Phe Ala Pro Gly Ile Trp Ser Asp Ala Gln Val Glu Ala
   65                  70                  75                  80
   tgg aag ccg atc gtg gcc ggt gtg cat gca aag ggc gga aag atc gtc       288
   Trp Lys Pro Ile Val Ala Gly Val His Ala Lys Gly Gly Lys Ile Val
                   85                  90                  95
   tgc cag ctc tgg cac atg ggc cgc atg gtc cac tcg tcc gtg acc gga       336
   Cys Gln Leu Trp His Met Gly Arg Met Val His Ser Ser Val Thr Gly
               100                 105                 110
   acg cag ccc gtc tcg tcc tcc gcc acc acg gcc ccc ggc gag gtc cat       384
   Thr Gln Pro Val Ser Ser Ser Ala Thr Thr Ala Pro Gly Glu Val His
           115                 120                 125
   acc tat gaa ggc aag aag ccg ttc gag cag gcc cgc gca atc gat gcc       432
   Thr Tyr Glu Gly Lys Lys Pro Phe Glu Gln Ala Arg Ala Ile Asp Ala
           130                 135                 140
   gcg gat atc agc cgt att ctg aac gac tat gag aac gcc gcc cgc aat       480
   Ala Asp Ile Ser Arg Ile Leu Asn Asp Tyr Glu Asn Ala Ala Arg Asn
   145                 150                 155                 160
   gcg atc cgc gcc ggc ttc gac ggg gtg cag atc cac gcc gcc aat ggc       528
   Ala Ile Arg Ala Gly Phe Asp Gly Val Gln Ile His Ala Ala Asn Gly
                   165                 170                 175
   tac ctc atc gac gag ttc ctg cga aac ggt acg aat cac cgc aca gat       576
   Tyr Leu Ile Asp Glu Phe Leu Arg Asn Gly Thr Asn His Arg Thr Asp
               180                 185                 190
   gaa tac ggc ggc gtc ccc gaa aac cgc atc cgt ttc ctg aag gaa gtc       624
   Glu Tyr Gly Gly Val Pro Glu Asn Arg Ile Arg Phe Leu Lys Glu Val
           195                 200                 205
   acc gag cgc gtg atc gct gcc atc ggt gcc gac cgc aca ggc gtg cgc       672
   Thr Glu Arg Val Ile Ala Ala Ile Gly Ala Asp Arg Thr Gly Val Arg
       210                 215                 220
   ctg tcc ccc aac ggc gat acg cag ggc tgc atc gac agc gca cct gag       720
   Leu Ser Pro Asn Gly Asp Thr Gln Gly Cys Ile Asp Ser Ala Pro Glu
   225                 230                 235                 240
   acg gtc ttt gtc ccg gcg gca aag ctg ctt cag gat ctg ggc gtg gcc       768
   Thr Val Phe Val Pro Ala Ala Lys Leu Leu Gln Asp Leu Gly Val Ala
                   245                 250                 255
   tgg ctc gaa ctg cgc gaa ccc ggc ccg aac ggc acc ttc ggc aag acg       816
   Trp Leu Glu Leu Arg Glu Pro Gly Pro Asn Gly Thr Phe Gly Lys Thr
               260                 265                 270
   gac cag ccc aaa ctg tcc ccg cag atc cgc aag gtg ttc ctg cgc ccg       864
   Asp Gln Pro Lys Leu Ser Pro Gln Ile Arg Lys Val Phe Leu Arg Pro
           275                 280                 285
   ctg gtg ctc aat cag gac tat acg ttc gag gca gca cag acc gcg ctg       912
   Leu Val Leu Asn Gln Asp Tyr Thr Phe Glu Ala Ala Gln Thr Ala Leu
       290                 295                 300
   gca gaa ggg aag gct gat gcg atc gcc ttc ggt cgc aag ttc atc tcg       960
   Ala Glu Gly Lys Ala Asp Ala Ile Ala Phe Gly Arg Lys Phe Ile Ser
   305                 310                 315                 320
   aac ccc gac ctg ccg gag cgc ttc gcc cgc ggc atc gcc ctg cag ccg      1008
   Asn Pro Asp Leu Pro Glu Arg Phe Ala Arg Gly Ile Ala Leu Gln Pro
                   325                 330                 335
   gat gat atg aaa acc tgg tac agt cag ggc ccc gaa gga tac acg gac      1056
   Asp Asp Met Lys Thr Trp Tyr Ser Gln Gly Pro Glu Gly Tyr Thr Asp
               340                 345                 350
   tac ccg tcc gcc acc tcc ggc ccc aac tga                              1086
   Tyr Pro Ser Ala Thr Ser Gly Pro Asn
           355                 360
<210> 4
   <211> 361
   <212> PRT
   <213> Gluconobacter oxydans
<400> 4
   Met Pro Thr Leu Phe Asp Pro Ile Asp Phe Gly Pro Ile His Ala Lys
   1 5 10 15
   Asn Arg Ile Val Met Ser Pro Leu Thr Arg Gly Arg Ala Asp Lys Glu
   20 25 30
   Ala Val Pro Thr Pro Ile Met Ala Glu Tyr Tyr Ala Gln Arg Ala Ser
   35 40 45
   Ala Gly Leu Ile Ile Thr Glu Ala Thr Gly Ile Ser Arg Glu Gly Leu
   50 55 60
   Gly Trp Pro Phe Ala Pro Gly Ile Trp Ser Asp Ala Gln Val Glu Ala
   65 70 75 80
   Trp Lys Pro Ile Val Ala Gly Val His Ala Lys Gly Gly Lys Ile Val
   85 90 95
   Cys Gln Leu Trp His Met Gly Arg Met Val His Ser Ser Val Thr Gly
   100 105 110
   Thr Gln Pro Val Ser Ser Ser Ala Thr Thr Ala Pro Gly Glu Val His
   115 120 125
   Thr Tyr Glu Gly Lys Lys Pro Phe Glu Gln Ala Arg Ala Ile Asp Ala
   130 135 140
   Ala Asp Ile Ser Arg Ile Leu Asn Asp Tyr Glu Asn Ala Ala Arg Asn
   145 150 155 160
   Ala Ile Arg Ala Gly Phe Asp Gly Val Gln Ile His Ala Ala Asn Gly
   165 170 175
   Tyr Leu Ile Asp Glu Phe Leu Arg Asn Gly Thr Asn His Arg Thr Asp
   180 185 190
   Glu Tyr Gly Gly Val Pro Glu Asn Arg Ile Arg Phe Leu Lys Glu Val
   195 200 205
   Thr Glu Arg Val Ile Ala Ala Ile Gly Ala Asp Arg Thr Gly Val Arg
   210 215 220
   Leu Ser Pro Asn Gly Asp Thr Gln Gly Cys Ile Asp Ser Ala Pro Glu
   225 230 235 240
   Thr Val Phe Val Pro Ala Ala Lys Leu Leu Gln Asp Leu Gly Val Ala
   245 250 255
   Trp Leu Glu Leu Arg Glu Pro Gly Pro Asn Gly Thr Phe Gly Lys Thr
   260 265 270
   Asp Gln Pro Lys Leu Ser Pro Gln Ile Arg Lys Val Phe Leu Arg Pro
   275 280 285
   Leu Val Leu Asn Gln Asp Tyr Thr Phe Glu Ala Ala Gln Thr Ala Leu
   290 295 300
   Ala Glu Gly Lys Ala Asp Ala Ile Ala Phe Gly Arg Lys Phe Ile Ser
   305 310 315 320
   Asn Pro Asp Leu Pro Glu Arg Phe Ala Arg Gly Ile Ala Leu Gln Pro
   325 330 335
   Asp Asp Met Lys Thr Trp Tyr Ser Gln Gly Pro Glu Gly Tyr Thr Asp
   340 345 350
   Tyr Pro Ser Ala Thr Ser Gly Pro Asn
   355 360
<210> 5
   <211> 840
   <212> DNA
   <213> Gluconobacter oxydans
<220>
   <221> CDS
   <222> (1)..(837)
<400> 5
   atg tcg tca cag gtt cca tcc gcc gag gcc cag acc gtg att tcc ttt        48
   Met Ser Ser Gln Val Pro Ser Ala Glu Ala Gln Thr Val Ile Ser Phe
   1               5                   10                  15
   cat gac ggt cac acc atg ccc cag atc ggg ctg ggc gtg tgg gaa acg        96
   His Asp Gly His Thr Met Pro Gln Ile Gly Leu Gly Val Trp Glu Thr
               20                  25                  30
   ccg ccg gat gag acg gcc gag gtc gtg aag gaa gcc gtg aag ctc ggt       144
   Pro Pro Asp Glu Thr Ala Glu Val Val Lys Glu Ala Val Lys Leu Gly
           35                  40                  45
   tac cgg tct gtc gat acg gcg cgt ctg tac aag aac gag gaa ggt gtc       192
   Tyr Arg Ser Val Asp Thr Ala Arg Leu Tyr Lys Asn Glu Glu Gly Val
       50                  55                  60
   ggc aaa ggt ctg gaa gac cat ccg gaa atc ttc ctg acg acc aag ctc       240
   Gly Lys Gly Leu Glu Asp His Pro Glu Ile Phe Leu Thr Thr Lys Leu
   65                  70                  75                  80
   tgg aat gac gag cag ggc tat gac agc acc ctg cgg gcg tat gaa gaa      288
   Trp Asn Asp Glu Gln Gly Tyr Asp Ser Thr Leu Arg Ala Tyr Glu Glu
                   85                  90                  95
   agc gcg cgc ctg ctg cgt cgt ccg gtg ctg gac ctg tat ctg atc cac      336
   Ser Ala Arg Leu Leu Arg Arg Pro Val Leu Asp Leu Tyr Leu Ile His
               100                 105                 110
   tgg ccg atg ccg gct cag ggg cag tat gtc gag acg tgg aag gca ctc      384
   Trp Pro Met Pro Ala Gln Gly Gln Tyr Val Glu Thr Trp Lys Ala Leu
           115                 120                 125
   gtc gag ctg aag aaa tcc ggt cgt gtg aag tcc atc ggc gtg tcc aat      432
   Val Glu Leu Lys Lys Ser Gly Arg Val Lys Ser Ile Gly Val Ser Asn
       130                 135                 140
   ttc gag tcg gag cat ctg gag cgg atc atg gat gcc acg ggt gtc gtg      480
   Phe Glu Ser Glu His Leu Glu Arg Ile Met Asp Ala Thr Gly Val Val
   145                 150                 155                 160
   ccg gtc gtc aac cag atc gag ctg cat ccc gat ttc cag cag cgc gcc      528
   Pro Val Val Asn Gln Ile Glu Leu His Pro Asp Phe Gln Gln Arg Ala
                   165                 170                 175
   ctg cgg gaa ttc cac gag aag cac aac atc cgc acc gag tcc tgg cgc      576
   Leu Arg Glu Phe His Glu Lys His Asn Ile Arg Thr Glu Ser Trp Arg
               180                 185                 190
   ccg ctg ggc aag ggg cgc gtc ctg agc gat gag cgg atc ggg aag atc      624
   Pro Leu Gly Lys Gly Arg Val Leu Ser Asp Glu Arg Ile Gly Lys Ile
           195                 200                 205
   gct gaa aag cac agc cgg act ccg gcg cag gtc gtg atc cgc tgg cat      672
   Ala Glu Lys His Ser Arg Thr Pro Ala Gln Val Val Ile Arg Trp His
       210                 215                 220
   ctt cag aat gga ctg atc gtc att ccg aaa tcg gtc aat ccc aag cgt      720
   Leu Gln Asn Gly Leu Ile Val Ile Pro Lys Ser Val Asn Pro Lys Arg
   225                 230                 235                 240
   ctg gct gaa aat ctg gat gtg ttc ggc ttc gtg ctg gat gcg gat gac      768
   Leu Ala Glu Asn Leu Asp Val Phe Gly Phe Val Leu Asp Ala Asp Asp
                   245                 250                 255
   atg cag gcc atc gaa cag atg gac cgc aag gat ggc cgg atg ggc gct      816
   Met Gln Ala Ile Glu Gln Met Asp Arg Lys Asp Gly Arg Met Gly Ala
               260                 265                 270
   gat ccg aat acg gcg aaa ttc tga                                      840
   Asp Pro Asn Thr Ala Lys Phe
           275
<210> 6
   <211> 279
   <212> PRT
   <213> Gluconobacter oxydans
<400> 6
   Met Ser Ser Gln Val Pro Ser Ala Glu Ala Gln Thr Val Ile Ser Phe
   1               5                   10                  15
   His Asp Gly His Thr Met Pro Gln Ile Gly Leu Gly Val Trp Glu Thr
               20                  25                  30
   Pro Pro Asp Glu Thr Ala Glu Val Val Lys Glu Ala Val Lys Leu Gly
           35                  40                  45
   Tyr Arg Ser Val Asp Thr Ala Arg Leu Tyr Lys Asn Glu Glu Gly Val
       50                  55                  60
   Gly Lys Gly Leu Glu Asp His Pro Glu Ile Phe Leu Thr Thr Lys Leu
   65                  70                  75                  80
   Trp Asn Asp Glu Gln Gly Tyr Asp Ser Thr Leu Arg Ala Tyr Glu Glu
                   85                  90                  95
   Ser Ala Arg Leu Leu Arg Arg Pro Val Leu Asp Leu Tyr Leu Ile His
               100                 105                 110
   Trp Pro Met Pro Ala Gln Gly Gln Tyr Val Glu Thr Trp Lys Ala Leu
           115                 120                 125
   Val Glu Leu Lys Lys Ser Gly Arg Val Lys Ser Ile Gly Val Ser Asn
       130                 135                 140
   Phe Glu Ser Glu His Leu Glu Arg Ile Met Asp Ala Thr Gly Val Val
   145                 150                 155                 160
   Pro Val Val Asn Gln Ile Glu Leu His Pro Asp Phe Gln Gln Arg Ala
                   165                 170                 175
   Leu Arg Glu Phe His Glu Lys His Asn Ile Arg Thr Glu Ser Trp Arg
               180                 185                 190
   Pro Leu Gly Lys Gly Arg Val Leu Ser Asp Glu Arg Ile Gly Lys Ile
           195                 200                 205
   Ala Glu Lys His Ser Arg Thr Pro Ala Gln Val Val Ile Arg Trp His
       210                 215                 220
   Leu Gln Asn Gly Leu Ile Val Ile Pro Lys Ser Val Asn Pro Lys Arg
   225                 230                 235                 240
   Leu Ala Glu Asn Leu Asp Val Phe Gly Phe Val Leu Asp Ala Asp Asp
                   245                 250                 255
   Met Gln Ala Ile Glu Gln Met Asp Arg Lys Asp Gly Arg Met Gly Ala
               260                 265                 270
   Asp Pro Asn Thr Ala Lys Phe
   275
<210> 7
   <211> 795
   <212> DNA
   <213> Gluconobacter oxydans
<220>
   <221> CDS
   <222> (1)..(795)
<400> 7
   atg ctg aac ctc gat ctg agc ggc cgc acc gcc ctc gtc acc ggc tcg      48
   Met Leu Asn Leu Asp Leu Ser Gly Arg Thr Ala Leu Val Thr Gly Ser
   1               5                   10                  15
   acc ggc ggg atc ggc ctg gcc atc gcc cgg aag ctg ggc gag gca gga      96
   Thr Gly Gly Ile Gly Leu Ala Ile Ala Arg Lys Leu Gly Glu Ala Gly
               20                  25                  30
   gcc aca gtc atc atc aac ggc cgc aag cag gaa ggc gtc gat agc gcc     144
   Ala Thr Val Ile Ile Asn Gly Arg Lys Gln Glu Gly Val Asp Ser Ala
           35                  40                  45
   ctt gag aag ctg gcc aag gcc atc ccg ggc ggc gcc ttc cgg agt gtc     192
   Leu Glu Lys Leu Ala Lys Ala Ile Pro Gly Gly Ala Phe Arg Ser Val
       50                  55                  60
   gtc gcc gat gtc gga acg gcc gaa ggc tgc aag gcg ctc ttt gaa gcc     240
   Val Ala Asp Val Gly Thr Ala Glu Gly Cys Lys Ala Leu Phe Glu Ala
   65                  70                  75                  80
   gaa agc agc gtg gat gtc ctc gtc aac aat gcc ggc atc ttc gga ccg     288
   Glu Ser Ser Val Asp Val Leu Val Asn Asn Ala Gly Ile Phe Gly Pro
                   85                  90                  95
   aat gat ttt ttc cag acg acg gat gaa agc tgg gag cac ctg ttc gag     336
   Asn Asp Phe Phe Gln Thr Thr Asp Glu Ser Trp Glu His Leu Phe Glu
               100                 105                 110
   gtc aac ctg ttc tcc ggc gtg cgc ctg tcc cgc gcc tat atg ccg ggc     384
   Val Asn Leu Phe Ser Gly Val Arg Leu Ser Arg Ala Tyr Met Pro Gly
           115                 120                 125
   atg aag gag cgc aac tgg ggc cgc gtg ctg ttc att gca tca gaa tcg     432
   Met Lys Glu Arg Asn Trp Gly Arg Val Leu Phe Ile Ala Ser Glu Ser
       130                 135                 140
   gca ctg aac atc ccg gtc gaa atg gtc gat tac ggc gtc agc aag acc     480
   Ala Leu Asn Ile Pro Val Glu Met Val Asp Tyr Gly Val Ser Lys Thr
   145                 150                 155                 160
   gcc atg ctg ggt ctg gcc cgc ggt ctg gcc aag ctg atg gcg ggc acg     528
   Ala Met Leu Gly Leu Ala Arg Gly Leu Ala Lys Leu Met Ala Gly Thr
                   165                 170                 175
   aat gtg acg gtg aat tcg gtc cta ccc ggc ccc acc ctg tcc gaa ggt     576
   Asn Val Thr Val Asn Ser Val Leu Pro Gly Pro Thr Leu Ser Glu Gly
               180                       185                       190
   gtc gag gac atg ctc aag gcc cag aac ccc gac agc acc cgc ccg gtc        624
   Val Glu Asp Met Leu Lys Ala Gln Asn Pro Asp Ser Thr Arg Pro Val
           195                     200                     205
   gcg gaa ctg gcg gcg gaa ttc gtg cag aaa cat cgc ccg agc aac atc        672
   Ala Glu Leu Ala Ala Glu Phe Val Gln Lys His Arg Pro Ser Asn Ile
       210                     215                     220
   ctg cgc cgc atg gcc acg gtt gat gaa gtg gcc aat ctc gtc gtc tat        720
   Leu Arg Arg Met Ala Thr Val Asp Glu Val Ala Asn Leu Val Val Tyr
   225                     230                     235                 240
   gcc gcc tcg cct ctg gcg tcc gcc aca acg ggc gcg gcc ctg cgc gtc        768
   Ala Ala Ser Pro Leu Ala Ser Ala Thr Thr Gly Ala Ala Leu Arg Val
                   245                     250                     255
   gat ggc ggc acc gtc gat acc atc tga                                    795
   Asp Gly Gly Thr Val Asp Thr Ile
               260
<210> 8
   <211> 264
   <212> PRT
   <213> Gluconobacter oxydans
<400> 8
   Met Leu Asn Leu Asp Leu Ser Gly Arg Thr Ala Leu Val Thr Gly Ser
   1               5                   10                  15
   Thr Gly Gly Ile Gly Leu Ala Ile Ala Arg Lys Leu Gly Glu Ala Gly
               20                  25                  30
   Ala Thr Val Ile Ile Asn Gly Arg Lys Gln Glu Gly Val Asp Ser Ala
           35                  40                  45
   Leu Glu Lys Leu Ala Lys Ala Ile Pro Gly Gly Ala Phe Arg Ser Val
       50                  55                  60
   Val Ala Asp Val Gly Thr Ala Glu Gly Cys Lys Ala Leu Phe Glu Ala
   65                  70                  75                  80
   Glu Ser Ser Val Asp Val Leu Val Asn Asn Ala Gly Ile Phe Gly Pro
                   85                  90                  95
   Asn Asp Phe Phe Gln Thr Thr Asp Glu Ser Trp Glu His Leu Phe Glu
               100                 105                 110
   Val Asn Leu Phe Ser Gly Val Arg Leu Ser Arg Ala Tyr Met Pro Gly
           115                 120                 125
   Met Lys Glu Arg Asn Trp Gly Arg Val Leu Phe Ile Ala Ser Glu Ser
       130                 135                 140
   Ala Leu Asn Ile Pro Val Glu Met Val Asp Tyr Gly Val Ser Lys Thr
   145                 150                 155                 160
   Ala Met Leu Gly Leu Ala Arg Gly Leu Ala Lys Leu Met Ala Gly Thr
                   165                 170                 175
   Asn Val Thr Val Asn Ser Val Leu Pro Gly Pro Thr Leu Ser Glu Gly
               180                 185                 190
   Val Glu Asp Met Leu Lys Ala Gln Asn Pro Asp Ser Thr Arg Pro Val
           195                 200                 205
   Ala Glu Leu Ala Ala Glu Phe Val Gln Lys His Arg Pro Ser Asn Ile
       210                 215                 220
   Leu Arg Arg Met Ala Thr Val Asp Glu Val Ala Asn Leu Val Val Tyr
   225                 230                 235                 240
   Ala Ala Ser Pro Leu Ala Ser Ala Thr Thr Gly Ala Ala Leu Arg Val
                   245                 250                 255
   Asp Gly Gly Thr Val Asp Thr Ile
               260
<210> 9
   <211> 999
   <212> DNA
   <213> Gluconobacter oxydans
<220>
   <221> CDS
   <222> (1)..(996)
<400> 9
   atg gca tcc gac acc atc cgc atc ccc ggc atc gac acg cct ctc tcc      48
   Met Ala Ser Asp Thr Ile Arg Ile Pro Gly Ile Asp Thr Pro Leu Ser
   1               5                   10                  15
   cgc gtc gcc ctc ggc acc tgg gcc atc ggc ggc tgg atg tgg ggc ggc      96
   Arg Val Ala Leu Gly Thr Trp Ala Ile Gly Gly Trp Met Trp Gly Gly
               20                  25                  30
   ccc gat gat gac aat ggc gtc cgc acc att cac gcg gcc ctc gat gaa     144
   Pro Asp Asp Asp Asn Gly Val Arg Thr Ile His Ala Ala Leu Asp Glu
           35                  40                  45
   ggc atc aac ctg atc gac acc gcc ccg gtc tac ggc ttc ggc cat tcc     192
   Gly Ile Asn Leu Ile Asp Thr Ala Pro Val Tyr Gly Phe Gly His Ser
       50                  55                  60
   gag gaa atc gtc ggc cgg gca ctc gcc gag aag ccc aac aag gcg cat     240
   Glu Glu Ile Val Gly Arg Ala Leu Ala Glu Lys Pro Asn Lys Ala His
   65                  70                  75                  80
   gtc gcc acg aaa ctc ggg ctc cac tgg gtt ggc gaa gac gaa aag aat      288
   Val Ala Thr Lys Leu Gly Leu His Trp Val Gly Glu Asp Glu Lys Asn
                   85                  90                  95
   atg aag gtc ttc cgg gac tcc cgc ccg gcc cgc atc cgc aag gaa gtc      336
   Met Lys Val Phe Arg Asp Ser Arg Pro Ala Arg Ile Arg Lys Glu Val
               100                 105                 110
   gag gac tcg ctc cgc cgt ctg cgt gtg gag acg atc gat ctc gag cag      384
   Glu Asp Ser Leu Arg Arg Leu Arg Val Glu Thr Ile Asp Leu Glu Gln
           115                 120                 125
   atc cac tgg ccc gac gac aaa acc ccg atc gac gag agc gcc cgc gag      432
   Ile His Trp Pro Asp Asp Lys Thr Pro Ile Asp Glu Ser Ala Arg Glu
       130                 135                 140
   ctt cag aaa ctc cat cag gac ggc aag atc cgt gcc ctc ggc gta agc      480
   Leu Gln Lys Leu His Gln Asp Gly Lys Ile Arg Ala Leu Gly Val Ser
   145                 150                 155                 160
   aac ttc tcg ccg gag cag atg gac atc ttc cgc gaa gtc gcc ccg ctc      528
   Asn Phe Ser Pro Glu Gln Met Asp Ile Phe Arg Glu Val Ala Pro Leu
                   165                 170                 175
   gcc acg atc cag ccc ccg ctg aac ctt ttc gaa cgc acc atc gag aag      576
   Ala Thr Ile Gln Pro Pro Leu Asn Leu Phe Glu Arg Thr Ile Glu Lys
               180                 185                 190
   gac atc ctg ccc tac gcc gaa aag cac aat gcc gtc gtt ctc gct tac      624
   Asp Ile Leu Pro Tyr Ala Glu Lys His Asn Ala Val Val Leu Ala Tyr
           195                 200                 205
   ggg gcc ctc tgc cgc ggc ctg ctg aca ggc aag atg aac cgc gat acg      672
   Gly Ala Leu Cys Arg Gly Leu Leu Thr Gly Lys Met Asn Arg Asp Thr
       210                 215                 220
   acc ttc ccg aag gat gac ctg cgt tcg aac gat ccg aag ttc cag aag      720
   Thr Phe Pro Lys Asp Asp Leu Arg Ser Asn Asp Pro Lys Phe Gln Lys
   225                 230                 235                 240
   ccg aat ttc gag aag tac ctc gca gcc atg gac gag ttc gaa aag ctt      768
   Pro Asn Phe Glu Lys Tyr Leu Ala Ala Met Asp Glu Phe Glu Lys Leu
                   245                 250                 255
   gcc gaa aag cgc ggc aag tct gtg atg gcc ttc gcg gtt cgc tgg gtt      816
   Ala Glu Lys Arg Gly Lys Ser Val Met Ala Phe Ala Val Arg Trp Val
               260                 265                 270
   ctg gat cag ggc ccg gta atc gcc ctg tgg ggt gcc cgc aag ccg ggt      864
   Leu Asp Gln Gly Pro Val Ile Ala Leu Trp Gly Ala Arg Lys Pro Gly
           275                 280                 285
   cag gtc tca ggc gta aag gac gtc ttc ggc tgg tcc ctg acc gac gaa      912
   Gln Val Ser Gly Val Lys Asp Val Phe Gly Trp Ser Leu Thr Asp Glu
       290                 295                 300
   gag aag aaa gcc gtg gac gac att ctg gct agg cat gtc ccc aat ccc      960
   Glu Lys Lys Ala Val Asp Asp Ile Leu Ala Arg His Val Pro Asn Pro
   305                 310                 315                 320
   atc gac ccg act ttc atg gcg ccc ccg gca cgg gac tga                    999
   Ile Asp Pro Thr Phe Met Ala Pro Pro Ala Arg Asp
                   325                     330
<210> 10
   <211> 332
   <212> PRT
   <213> Gluconobacter oxydans
<400> 10
   Met Ala Ser Asp Thr Ile Arg Ile Pro Gly Ile Asp Thr Pro Leu Ser
   1               5                   10                  15
   Arg Val Ala Leu Gly Thr Trp Ala Ile Gly Gly Trp Met Trp Gly Gly
               20                  25                  30
   Pro Asp Asp Asp Asn Gly Val Arg Thr Ile His Ala Ala Leu Asp Glu
           35                  40                  45
   Gly Ile Asn Leu Ile Asp Thr Ala Pro Val Tyr Gly Phe Gly His Ser
       50                  55                  60
   Glu Glu Ile Val Gly Arg Ala Leu Ala Glu Lys Pro Asn Lys Ala His
   65                  70                  75                  80
   Val Ala Thr Lys Leu Gly Leu His Trp Val Gly Glu Asp Glu Lys Asn
                   85                  90                  95
   Met Lys Val Phe Arg Asp Ser Arg Pro Ala Arg Ile Arg Lys Glu Val
               100                 105                 110
   Glu Asp Ser Leu Arg Arg Leu Arg Val Glu Thr Ile Asp Leu Glu Gln
           115                 120                 125
   Ile His Trp Pro Asp Asp Lys Thr Pro Ile Asp Glu Ser Ala Arg Glu
       130                 135                 140
   Leu Gln Lys Leu His Gln Asp Gly Lys Ile Arg Ala Leu Gly Val Ser
   145                 150                 155                 160
   Asn Phe Ser Pro Glu Gln Met Asp Ile Phe Arg Glu Val Ala Pro Leu
                   165                 170                 175
   Ala Thr Ile Gln Pro Pro Leu Asn Leu Phe Glu Arg Thr Ile Glu Lys
               180                 185                 190
   Asp Ile Leu Pro Tyr Ala Glu Lys His Asn Ala Val Val Leu Ala Tyr
           195                 200                 205
   Gly Ala Leu Cys Arg Gly Leu Leu Thr Gly Lys Met Asn Arg Asp Thr
       210                 215                 220
   Thr Phe Pro Lys Asp Asp Leu Arg Ser Asn Asp Pro Lys Phe Gln Lys
   225                 230                 235                 240
   Pro Asn Phe Glu Lys Tyr Leu Ala Ala Met Asp Glu Phe Glu Lys Leu
                   245                 250                 255
   Ala Glu Lys Arg Gly Lys Ser Val Met Ala Phe Ala Val Arg Trp Val
               260                 265                 270
   Leu Asp Gln Gly Pro Val Ile Ala Leu Trp Gly Ala Arg Lys Pro Gly
           275                 280                 285
   Gln Val Ser Gly Val Lys Asp Val Phe Gly Trp Ser Leu Thr Asp Glu
       290                 295                 300
   Glu Lys Lys Ala Val Asp Asp Ile Leu Ala Arg His Val Pro Asn Pro
   305                 310                 315                 320
   Ile Asp Pro Thr Phe Met Ala Pro Pro Ala Arg Asp
                   325                 330
<210> 11
   <211> 591
   <212> DNA
   <213> Gluconobacter oxydans
<220>
   <221> CDS
   <222> (1)..(588)
<400> 11
   atg acg aaa att ctt ctc ctt aac ggc ggc aag gtc ttc gcc cat tcc      48
   Met Thr Lys Ile Leu Leu Leu Asn Gly Gly Lys Val Phe Ala His Ser
   1               5                   10                  15
   cac gga cgg ctg aac gac acg ctt cag gcg ctg gcg gcg gac atc ctg      96
   His Gly Arg Leu Asn Asp Thr Leu Gln Ala Leu Ala Ala Asp Ile Leu
               20                  25                  30
   ttc aag aac ggc cat gag atc cgc cag acg aag atc gat gac ggc tat     144
   Phe Lys Asn Gly His Glu Ile Arg Gln Thr Lys Ile Asp Asp Gly Tyr
           35                  40                  45
   gac gtg gaa cag gaa gtc gaa agc ttc atc tgg gcc gat ctg atc atc     192
   Asp Val Glu Gln Glu Val Glu Ser Phe Ile Trp Ala Asp Leu Ile Ile
       50                  55                  60
   tat cag tgc ccg ggc tgg tgg atg ggg ctg ccc tgg atc gtc aag aaa     240
   Tyr Gln Cys Pro Gly Trp Trp Met Gly Leu Pro Trp Ile Val Lys Lys
   65                  70                  75                  80
   tac atc gac gaa gtc atg acg gcc ggt cac ggc aag ctc tat gcc aat     288
   Tyr Ile Asp Glu Val Met Thr Ala Gly His Gly Lys Leu Tyr Ala Asn
                   85                  90                  95
   gac ggc cgc agc cgg gcc cat ccg gag cgc aaa tat ggc tcg ggc ggt     336
   Asp Gly Arg Ser Arg Ala His Pro Glu Arg Lys Tyr Gly Ser Gly Gly
               100                 105                 110
   ctg gcg cag ggt aag cgc tat atg ctg tca acg acc tgg aat gcg ccg     384
   Leu Ala Gln Gly Lys Arg Tyr Met Leu Ser Thr Thr Trp Asn Ala Pro
           115                 120                 125
   gca gag gct ttc acg gac ccg gcc cag ttc ttt gaa ggg cgg ggt atc     432
   Ala Glu Ala Phe Thr Asp Pro Ala Gln Phe Phe Glu Gly Arg Gly Ile
       130                 135                 140
   gac ggg gtg tat ttc ccg ttc cac aag gcc cac gag ttc ctg ggc atg     480
   Asp Gly Val Tyr Phe Pro Phe His Lys Ala His Glu Phe Leu Gly Met
   145                 150                 155                 160
   tcc gcg ctc ccg acc ttc ctt gcg acg gac gtg ata aag gcg ccg aat     528
   Ser Ala Leu Pro Thr Phe Leu Ala Thr Asp Val Ile Lys Ala Pro Asn
                   165                 170                 175
   gtg gag gca aca ctc gca gcc tac cgc cag cat ctg cag gac gtc atc     576
   Val Glu Ala Thr Leu Ala Ala Tyr Arg Gln His Leu Gln Asp Val Ile
               180                 185                 190
   ggt acg gcg tcc tga                                                 591
   Gly Thr Ala Ser
           195
<210> 12
   <211> 196
   <212> PRT
   <213> Gluconobacter oxydans
<400> 12
   Met Thr Lys Ile Leu Leu Leu Asn Gly Gly Lys Val Phe Ala His Ser
   1               5                   10                  15
   His Gly Arg Leu Asn Asp Thr Leu Gln Ala Leu Ala Ala Asp Ile Leu
               20                  25                  30
   Phe Lys Asn Gly His Glu Ile Arg Gln Thr Lys Ile Asp Asp Gly Tyr
           35                  40                  45
   Asp Val Glu Gln Glu Val Glu Ser Phe Ile Trp Ala Asp Leu Ile Ile
       50                  55                  60
   Tyr Gln Cys Pro Gly Trp Trp Met Gly Leu Pro Trp Ile Val Lys Lys
   65                  70                  75                  80
   Tyr Ile Asp Glu Val Met Thr Ala Gly His Gly Lys Leu Tyr Ala Asn
                   85                  90                  95
   Asp Gly Arg Ser Arg Ala His Pro Glu Arg Lys Tyr Gly Ser Gly Gly
               100                 105                 110
   Leu Ala Gln Gly Lys Arg Tyr Met Leu Ser Thr Thr Trp Asn Ala Pro
           115                 120                 125
   Ala Glu Ala Phe Thr Asp Pro Ala Gln Phe Phe Glu Gly Arg Gly Ile
       130                 135                 140
   Asp Gly Val Tyr Phe Pro Phe His Lys Ala His Glu Phe Leu Gly Met
   145                 150                 155                 160
   Ser Ala Leu Pro Thr Phe Leu Ala Thr Asp Val Ile Lys Ala Pro Asn
                   165                 170                 175
   Val Glu Ala Thr Leu Ala Ala Tyr Arg Gln His Leu Gln Asp Val Ile
               180                 185                 190
   Gly Thr Ala Ser
           195
<210> 13
   <211> 1074
   <212> DNA
   <213> Gluconobacter oxydans
<220>
   <221> CDS
   <222> (1) .. (1074)
<400> 13
   atg acc agc ctg ttt gag ccg att gaa ctg gga agc att tac gcc aaa       48
   Met Thr Ser Leu Phe Glu Pro Ile Glu Leu Gly Ser Ile Tyr Ala Lys
   1               5                   10                  15
   aac aga att ctc atg gcg ccg ctg aca cgc ggt cgg ggc acc cgt gat       96
   Asn Arg Ile Leu Met Ala Pro Leu Thr Arg Gly Arg Gly Thr Arg Asp
               20                  25                  30
   cat gtg ccc acc ccc atc atg gcc gaa tat tac gcg caa cgg gcc gga      144
   His Val Pro Thr Pro Ile Met Ala Glu Tyr Tyr Ala Gln Arg Ala Gly
           35                  40                  45
   gcc ggt ctg atc atc tca gaa gcg acc ggg atc agc cgc gaa ggt ctt      192
   Ala Gly Leu Ile Ile Ser Glu Ala Thr Gly Ile Ser Arg Glu Gly Leu
       50                  55                  60
   ggc tgg ccg tat gcg ccg ggc ctg tgg tcg cag gaa cag gtg gaa gcc      240
   Gly Trp Pro Tyr Ala Pro Gly Leu Trp Ser Gln Glu Gln Val Glu Ala
   65                  70                  75                  80
   tgg aag ccc atc acc gcc gca gtt cac gcc aag ggc gga aag att gtg      288
   Trp Lys Pro Ile Thr Ala Ala Val His Ala Lys Gly Gly Lys Ile Val
                   85                  90                  95
   gcc cag ctc tgg cat atg ggt cgg atg gtt cat tcc agc gtg acg ggc      336
   Ala Gln Leu Trp His Met Gly Arg Met Val His Ser Ser Val Thr Gly
               100                 105                 110
   cag caa cct gtg tcc tgc tcg gcg aca aaa gcg cct gaa gcc ctc cat      384
   Gln Gln Pro Val Ser Cys Ser Ala Thr Lys Ala Pro Glu Ala Leu His
           115                 120                 125
   acg tac gat ggc aag cag gct ccc gaa gtc gcc cgc cct ctc acc aag      432
   Thr Tyr Asp Gly Lys Gln Ala Pro Glu Val Ala Arg Pro Leu Thr Lys
       130                 135                 140
   gag gac att gcc cgc atc ctg aac gac tac gaa aat gct gct cgc aat      480
   Glu Asp Ile Ala Arg Ile Leu Asn Asp Tyr Glu Asn Ala Ala Arg Asn
   145                 150                 155                 160
   gcc ctt cag gca ggc ttt gac ggt gtg cag att cat gcc gcc aac ggt      528
   Ala Leu Gln Ala Gly Phe Asp Gly Val Gln Ile His Ala Ala Asn Gly
                   165                 170                 175
   tat ctg atc gac gaa ttt ctg cgg gac ggc acc aat cat cgt tcc gac      576
   Tyr Leu Ile Asp Glu Phe Leu Arg Asp Gly Thr Asn His Arg Ser Asp
               180                 185                 190
   gaa tat ggc ggt tcg ccg gaa aac cgc atc cgc ttc ctg cgt gaa gtc      624
   Glu Tyr Gly Gly Ser Pro Glu Asn Arg Ile Arg Phe Leu Arg Glu Val
           195                 200                 205
   acc gaa cgc gtg atc gca acg att ggc gcg cac aaa acg tca gta agg      672
   Thr Glu Arg Val Ile Ala Thr Ile Gly Ala His Lys Thr Ser Val Arg
       210                 215                 220
   ctg tcc ccc aat ggc gat acg gtg ggc tgc atc gac agt cat cca gag      720
   Leu Ser Pro Asn Gly Asp Thr Val Gly Cys Ile Asp Ser His Pro Glu
   225                 230                 235                 240
   cag gtt ttt gtg ccg gcc gca aag ctg ctg aat gac ctc gat atc gct      768
   Gln Val Phe Val Pro Ala Ala Lys Leu Leu Asn Asp Leu Asp Ile Ala
                   245                 250                 255
   ttc ctt gaa ctg cgc gag ccc gga ccg aac ggc acg ttc ggc aag acc      816
   Phe Leu Glu Leu Arg Glu Pro Gly Pro Asn Gly Thr Phe Gly Lys Thr
               260                 265                 270
   gac cag ccc aag ctg cat ggt ccg atc cgc gaa gtc ttc agg aag ccg      864
   Asp Gln Pro Lys Leu His Gly Pro Ile Arg Glu Val Phe Arg Lys Pro
           275                 280                 285
   ctg gtt ctg aat cag gac tac aca cgg gaa gaa gcg atc gag aca gtc      912
   Leu Val Leu Asn Gln Asp Tyr Thr Arg Glu Glu Ala Ile Glu Thr Val
       290                 295                 300
   gct acc ggt gtt gcg gac gcc att tcg ttt ggt cgg cct ttc ctc gcc      960
   Ala Thr Gly Val Ala Asp Ala Ile Ser Phe Gly Arg Pro Phe Leu Ala
   305                 310                 315                 320
   aat ccg gac ctc gtg cgt cgt ctg gaa gac aat ctg ccc cag aac aag     1008
   Asn Pro Asp Leu Val Arg Arg Leu Glu Asp Asn Leu Pro Gln Asn Lys
                   325                 330                 335
   gac gat atc cgc acc tgg tac tcg cag ggt gca gaa ggc tat acg gat     1056
   Asp Asp Ile Arg Thr Trp Tyr Ser Gln Gly Ala Glu Gly Tyr Thr Asp
               340                 345                 350
   tat ccg ctt gct cgc tga                                            1074
   Tyr Pro Leu Ala Arg
           355
<210> 14
   <211> 357
   <212> PRT
   <213> Gluconobacter oxydans
<400> 14
   Met Thr Ser Leu Phe Glu Pro Ile Glu Leu Gly Ser Ile Tyr Ala Lys
   1               5                   10                  15
   Asn Arg Ile Leu Met Ala Pro Leu Thr Arg Gly Arg Gly Thr Arg Asp
               20                  25                  30
   His Val Pro Thr Pro Ile Met Ala Glu Tyr Tyr Ala Gln Arg Ala Gly
           35                  40                  45
   Ala Gly Leu Ile Ile Ser Glu Ala Thr Gly Ile Ser Arg Glu Gly Leu
       50                  55                  60
   Gly Trp Pro Tyr Ala Pro Gly Leu Trp Ser Gln Glu Gln Val Glu Ala
   65                  70                  75                  80
   Trp Lys Pro Ile Thr Ala Ala Val His Ala Lys Gly Gly Lys Ile Val
                   85                  90                  95
   Ala Gln Leu Trp His Met Gly Arg Met Val His Ser Ser Val Thr Gly
               100                 105                 110
   Gln Gln Pro Val Ser Cys Ser Ala Thr Lys Ala Pro Glu Ala Leu His
           115                 120                 125
   Thr Tyr Asp Gly Lys Gln Ala Pro Glu Val Ala Arg Pro Leu Thr Lys
       130                 135                 140
   Glu Asp Ile Ala Arg Ile Leu Asn Asp Tyr Glu Asn Ala Ala Arg Asn
   145                 150                 155                 160
   Ala Leu Gln Ala Gly Phe Asp Gly Val Gln Ile His Ala Ala Asn Gly
                   165                 170                 175
   Tyr Leu Ile Asp Glu Phe Leu Arg Asp Gly Thr Asn His Arg Ser Asp
               180                 185                 190
   Glu Tyr Gly Gly Ser Pro Glu Asn Arg Ile Arg Phe Leu Arg Glu Val
       195                 200                 205
   Thr Glu Arg Val Ile Ala Thr Ile Gly Ala His Lys Thr Ser Val Arg
       210                 215                 220
   Leu Ser Pro Asn Gly Asp Thr Val Gly Cys Ile Asp Ser His Pro Glu
   225                 230                 235                 240
   Gln Val Phe Val Pro Ala Ala Lys Leu Leu Asn Asp Leu Asp Ile Ala
                   245                 250                 255
   Phe Leu Glu Leu Arg Glu Pro Gly Pro Asn Gly Thr Phe Gly Lys Thr
               260                 265                 270
   Asp Gln Pro Lys Leu His Gly Pro Ile Arg Glu Val Phe Arg Lys Pro
           275                 280                 285
   Leu Val Leu Asn Gln Asp Tyr Thr Arg Glu Glu Ala Ile Glu Thr Val
       290                 295                 300
   Ala Thr Gly Val Ala Asp Ala Ile Ser Phe Gly Arg Pro Phe Leu Ala
   305                 310                 315                 320
   Asn Pro Asp Leu Val Arg Arg Leu Glu Asp Asn Leu Pro Gln Asn Lys
                   325                 330                 335
   Asp Asp Ile Arg Thr Trp Tyr Ser Gln Gly Ala Glu Gly Tyr Thr Asp
               340                 345                 350
   Tyr Pro Leu Ala Arg
           355
<210> 15
   <211> 1392
   <212> DNA
   <213> Gluconobacter oxydans
<220>
   <221> CDS
   <222> (1) .. (1389)
<400> 15
   atg gct tac gct acg atc aac cct tat acc ggt gag acg ctc aag acc      48
   Met Ala Tyr Ala Thr Ile Asn Pro Tyr Thr Gly Glu Thr Leu Lys Thr
   1               5                   10                  15
   ttc ccg gaa gca acc gac cag gaa gtc cag acc gcg ctg acc gaa gcc      96
   Phe Pro Glu Ala Thr Asp Gln Glu Val Gln Thr Ala Leu Thr Glu Ala
               20                  25                  30
   tat gat gcg ttc aag agc tgg cgt cat acg ccg ttc gcc gag cgc gca      144
   Tyr Asp Ala Phe Lys Ser Trp Arg His Thr Pro Phe Ala Glu Arg Ala
           35                  40                  45
   aag atc atg acg gca gcg gcg aac att ctt cgc cgt gat atc gac aag      192
   Lys Ile Met Thr Ala Ala Ala Asn Ile Leu Arg Arg Asp Ile Asp Lys
       50                  55                  60
   tat tcc aag ctt ctg acg ctc gag atg ggc aag atc tat gcc gaa gcc      240
   Tyr Ser Lys Leu Leu Thr Leu Glu Met Gly Lys Ile Tyr Ala Glu Ala
   65                  70                  75                  80
   aag gca gag acg atc ctg tcc gcc gag atc ttc gaa tat tat gcc aag      288
   Lys Ala Glu Thr Ile Leu Ser Ala Glu Ile Phe Glu Tyr Tyr Ala Lys
                   85                  90                  95
   aac gcc gaa gaa ctg ctg aaa gcc gag aag ctg ccg gtt gcg gat ccg      336
   Asn Ala Glu Glu Leu Leu Lys Ala Glu Lys Leu Pro Val Ala Asp Pro
               100                 105                 110
   aag gaa ggc gag gcc gtc ctg atc cac cag ccg cag ggc att gtg ttc      384
   Lys Glu Gly Glu Ala Val Leu Ile His Gln Pro Gln Gly Ile Val Phe
           115                 120                 125
   gcg atc gag ccg tgg aac ttc ccg ttc tac cag atc gcc cgc atc atc      432
   Ala Ile Glu Pro Trp Asn Phe Pro Phe Tyr Gln Ile Ala Arg Ile Ile
       130                 135                 140
   gcg ccg cag ctc tcc gcc ggt aac acg gtc att ctc aag cac gcc tcc      480
   Ala Pro Gln Leu Ser Ala Gly Asn Thr Val Ile Leu Lys His Ala Ser
   145                 150                 155                 160
   aac gtg ccg cag tgt gct gct gcg ttc gat gac ctg atg aag gaa gcc      528
   Asn Val Pro Gln Cys Ala Ala Ala Phe Asp Asp Leu Met Lys Glu Ala
                   165                 170                 175
   ggt ctg ccg aag ggt gga ttc cgc aac ctg tat gct gcc cgt cat cac      576
   Gly Leu Pro Lys Gly Gly Phe Arg Asn Leu Tyr Ala Ala Arg His His
               180                 185                 190
   acc gag acg atc ctc aag gac ccg cgc gtc tgt ggc gtg gcc ctg acg      624
   Thr Glu Thr Ile Leu Lys Asp Pro Arg Val Cys Gly Val Ala Leu Thr
           195                 200                 205
   ggt tcg gaa ggt gct ggc gct gtt gtg gcc ggg att gcg gca cag gcg      672
   Gly Ser Glu Gly Ala Gly Ala Val Val Ala Gly Ile Ala Ala Gln Ala
       210                 215                 220
   ctg aag aag tcc acg atg gag ctc ggg ggt tcc gat gcg ttc ctc gtt      720
   Leu Lys Lys Ser Thr Met Glu Leu Gly Gly Ser Asp Ala Phe Leu Val
   225                 230                 235                 240
   ctg gac gat gcg gat ctg gaa aag acc gcg aag tgg gct gct ttc ggc      768
   Leu Asp Asp Ala Asp Leu Glu Lys Thr Ala Lys Trp Ala Ala Phe Gly
                   245                 250                 255
   cgt cac tgg aat gcc ggt cag gtc tgc gtg tcc gca aag cgt ctg atc      816
   Arg His Trp Asn Ala Gly Gln Val Cys Val Ser Ala Lys Arg Leu Ile
               260                 265                 270
   gtg gcg gat gcc gtg tat gat cgc ttc gtc gag ctg tac aag gcc gaa      864
   Val Ala Asp Ala Val Tyr Asp Arg Phe Val Glu Leu Tyr Lys Ala Glu
           275                 280                 285
   gtc gcc aag ctg atc ccg ggt gat ccg atg gac ccg aag acc acg ctg      912
   Val Ala Lys Leu Ile Pro Gly Asp Pro Met Asp Pro Lys Thr Thr Leu
       290                 295                 300
   gct ccg ctg tcc tcg cag aag gct gct gat gat ctg aag gct cag gtc      960
   Ala Pro Leu Ser Ser Gln Lys Ala Ala Asp Asp Leu Lys Ala Gln Val
   305                 310                 315                 320
   gag gaa gcc aag aag cac ggt gcc gtc gtc gag gcc att ggt ccg tcc     1008
   Glu Glu Ala Lys Lys His Gly Ala Val Val Glu Ala Ile Gly Pro Ser
                   325                 330                 335
   gta ccg gag aag ggt gcc ttc ttc cag ccg ctg ctg atg acc aac ctg     1056
   Val Pro Glu Lys Gly Ala Phe Phe Gln Pro Leu Leu Met Thr Asn Leu
               340                 345                 350
   cac gag aac aat gaa gcc cgt ctg tgg gag ttc ttt ggt ccg gtg acg     1104
   His Glu Asn Asn Glu Ala Arg Leu Trp Glu Phe Phe Gly Pro Val Thr
           355                 360                 365
   cag ctt tat cgt gcc aag gac gat gcg gat gcc atc cgt atc gcc aat     1152
   Gln Leu Tyr Arg Ala Lys Asp Asp Ala Asp Ala Ile Arg Ile Ala Asn
       370                 375                 380
   gac tcg ccg ttc ggt ctg ggt ggt tcg gtc ttc acg aag gac acc cag     1200
   Asp Ser Pro Phe Gly Leu Gly Gly Ser Val Phe Thr Lys Asp Thr Gln
   385                 390                 395                 400
   cgt ggc gtg aag gtt gcc gag cag atc tat acg ggc atg gtc tat atc     1248
   Arg Gly Val Lys Val Ala Glu Gln Ile Tyr Thr Gly Met Val Tyr Ile
                   405                 410                 415
   aac cat ccg acg atg gtg aag gct gat ctg ccg ttc ggt ggc gtg gcc     1296
   Asn His Pro Thr Met Val Lys Ala Asp Leu Pro Phe Gly Gly Val Ala
               420                 425                 430
   cgt tcg ggt tat ggc cgt gag ctg atc ggt ctg ggt atc aag gaa ttc     1344
   Arg Ser Gly Tyr Gly Arg Glu Leu Ile Gly Leu Gly Ile Lys Glu Phe
           435                 440                 445
   gtc aat cac aag ctg atc gac gtt gtt gat atc gac gcg ccg ttc tga     1392
   Val Asn His Lys Leu Ile Asp Val Val Asp Ile Asp Ala Pro Phe
       450                 455                 460
<210> 16
   <211> 463
   <212> PRT
   <213> Gluconobacter oxydans
<400> 16
   Met Ala Tyr Ala Thr Ile Asn Pro Tyr Thr Gly Glu Thr Leu Lys Thr
   1               5                   10                  15
   Phe Pro Glu Ala Thr Asp Gln Glu Val Gln Thr Ala Leu Thr Glu Ala
               20                  25                  30
   Tyr Asp Ala Phe Lys Ser Trp Arg His Thr Pro Phe Ala Glu Arg Ala
           35                  40                  45
   Lys Ile Met Thr Ala Ala Ala Asn Ile Leu Arg Arg Asp Ile Asp Lys
       50                  55                  60
   Tyr Ser Lys Leu Leu Thr Leu Glu Met Gly Lys Ile Tyr Ala Glu Ala
   65                  70                  75                  80
   Lys Ala Glu Thr Ile Leu Ser Ala Glu Ile Phe Glu Tyr Tyr Ala Lys
                   85                  90                  95
   Asn Ala Glu Glu Leu Leu Lys Ala Glu Lys Leu Pro Val Ala Asp Pro
               100                 105                 110
   Lys Glu Gly Glu Ala Val Leu Ile His Gln Pro Gln Gly Ile Val Phe
           115                 120                 125
   Ala Ile Glu Pro Trp Asn Phe Pro Phe Tyr Gln Ile Ala Arg Ile Ile
       130                 135                 140
   Ala Pro Gln Leu Ser Ala Gly Asn Thr Val Ile Leu Lys His Ala Ser
   145                 150                 155                 160
   Asn Val Pro Gln Cys Ala Ala Ala Phe Asp Asp Leu Met Lys Glu Ala
                   165                 170                 175
   Gly Leu Pro Lys Gly Gly Phe Arg Asn Leu Tyr Ala Ala Arg His His
               180                 185                 190
   Thr Glu Thr Ile Leu Lys Asp Pro Arg Val Cys Gly Val Ala Leu Thr
           195                 200                 205
   Gly Ser Glu Gly Ala Gly Ala Val Val Ala Gly Ile Ala Ala Gln Ala
       210                 215                 220
   Leu Lys Lys Ser Thr Met Glu Leu Gly Gly Ser Asp Ala Phe Leu Val
   225                 230                 235                 240
   Leu Asp Asp Ala Asp Leu Glu Lys Thr Ala Lys Trp Ala Ala Phe Gly
                   245                 250                 255
   Arg His Trp Asn Ala Gly Gln Val Cys Val Ser Ala Lys Arg Leu Ile
               260                 265                 270
   Val Ala Asp Ala Val Tyr Asp Arg Phe Val Glu Leu Tyr Lys Ala Glu
           275                 280                 285
   Val Ala Lys Leu Ile Pro Gly Asp Pro Met Asp Pro Lys Thr Thr Leu
           290                 295                 300
   Ala Pro Leu Ser Ser Gln Lys Ala Ala Asp Asp Leu Lys Ala Gln Val
   305                 310                 315                 320
   Glu Glu Ala Lys Lys His Gly Ala Val Val Glu Ala Ile Gly Pro Ser
                   325                 330                 335
   Val Pro Glu Lys Gly Ala Phe Phe Gln Pro Leu Leu Met Thr Asn Leu
               340                 345                 350
   His Glu Asn Asn Glu Ala Arg Leu Trp Glu Phe Phe Gly Pro Val Thr
           355                 360                 365
   Gln Leu Tyr Arg Ala Lys Asp Asp Ala Asp Ala Ile Arg Ile Ala Asn
       370                 375                 380
   Asp Ser Pro Phe Gly Leu Gly Gly Ser Val Phe Thr Lys Asp Thr Gln
   385                 390                 395                 400
   Arg Gly Val Lys Val Ala Glu Gln Ile Tyr Thr Gly Met Val Tyr Ile
                   405                 410                 415
   Asn His Pro Thr Met Val Lys Ala Asp Leu Pro Phe Gly Gly Val Ala
               420                 425                 430
   Arg Ser Gly Tyr Gly Arg Glu Leu Ile Gly Leu Gly Ile Lys Glu Phe
           435                 440                 445
   Val Asn His Lys Leu Ile Asp Val Val Asp Ile Asp Ala Pro Phe
       450                 455                 460
<210> 17
   <211> 40
   <212> DNA
   <213> Artificial
<220>
   <223> primer
<400> 17
   atggtagaag acaagcgccg ctgatacaat gctcgccgcc 40
<210> 18
   <211> 40
   <212> DNA
   <213> Artificial
<220>
   <223> primer
<400> 18
   atggtagaag acaatatcag gaccggaagt cgagcactgt 40
<210> 19
   <211> 40
   <212> DNA
   <213> Artificial
<220>
   <223> primer
<400> 19
   atggtaggtc tcagcgcccc aaccctgttc gatcccattg 40
<210> 20
   <211> 37
   <212> DNA
   <213> Artificial
<220>
   <223> primer
<400> 20
   atggtaggtc tcatatcagt tggggccgga ggtggcg 37
<210> 21
   <211> 39
   <212> DNA
   <213> Artificial
<220>
   <223> primer
<400> 21
   atggtaggtc tcagcgcctc gtcacaggtt ccatccgcc 39
<210> 22
   <211> 41
   <212> DNA
   <213> Artificial
<220>
   <223> primer
<400> 22
   atggtaggtc tcatatcaga atttcgccgt attcggatca g 41
<210> 23
   <211> 38
   <212> DNA
   <213> Artificial
<220>
   <223> primer
<400> 23
   atggtaggtc tcaaatgctg aacctcgatc tgagcggc 38
<210> 24
   <211> 39
   <212> DNA
   <213> Artificial
<220>
   <223> primer
<400> 24
   atggtaggtc tcagcgctga tggtatcgac ggtgccgcc 39
<210> 25
   <211> 39
   <212> DNA
   <213> Artificial
<220>
   <223> primer
<400> 25
   atggtacgtc tcagcgccgc atccgacacc atccgcatc 39
<210> 26
   <211> 36
   <212> DNA
   <213> Artificial
<220>
   <223> primer
<400> 26
   atggtacgtc tcatatcagt cccgtgccgg gggcgc 36
<210> 27
   <211> 42
   <212> DNA
   <213> Artificial
<220>
   <223> primer
<400> 27
   atggtaggtc tcagcgccac gaaaattctt ctccttaacg gc 42
<210> 28
   <211> 39
   <212> DNA
   <213> Artificial
<220>
   <223> primer
<400> 28
   atggtaggtc tcatatcagg acgccgtacc gatgacgtc 39
<210> 29
   <211> 40
   <212> DNA
   <213> Artificial
<220>
   <223> primer
<400> 29
   atggtaggtc tcagcgccac cagcctgttt gagccgattg 40
<210> 30
   <211> 40
   <212> DNA
   <213> Artificial
<220>
   <223> primer
<400> 30
   atggtaggtc tcatatcagc gagcaagcgg ataatccgta 40
<210> 31
   <211> 41
   <212> DNA
   <213> Artificial
<220>
   <223> primer
<400> 31
   atggtaggtc tcagcgccgc ttacgctacg atcaaccctt a 41
<210> 32
   <211> 40
   <212> DNA
   <213> Artificial
<220>
   <223> primer
<400> 32
   atggtaggtc tcatatcaga acggcgcgtc gatatcaaca 40
<210> 33
   <211> 3239
   <212> DNA
   <213> Artificial
<220>
   <223> vector pASK-IBA5
<400> 33
   ccatcgaatg gccagatgat taattcctaa tttttgttga cactctatca ttgatagagt      60
   tattttacca ctccctatca gtgatagaga aaagtgaaat gaatagttcg acaaaaatct     120
   agataacgag ggcaaaaaat ggctagctgg agccacccgc agttcgaaaa aggcgccgag     180
   accgcggtcc cgaattcgag ctcggtaccc ggggatccct cgaggtcgac ctgcaggggg     240
   accatggtct ctgatatcta actaagcttg acctgtgaag tgaaaaatgg cgcacattgt 300
   gcgacatttt ttttgtctgc cgtttaccgc tactgcgtca cggatctcca cgcgccctgt 360
   agcggcgcat taagcgcggc gggtgtggtg gttacgcgca gcgtgaccgc tacacttgcc 420
   agcgccctag cgcccgctcc tttcgctttc ttcccttcct ttctcgccac gttcgccggc 480
   tttccccgtc aagctctaaa tcgggggctc cctttagggt tccgatttag tgctttacgg 540
   cacctcgacc ccaaaaaact tgattagggt gatggttcac gtagtgggcc atcgccctga 600
   tagacggttt ttcgcccttt gacgttggag tccacgttct ttaatagtgg actcttgttc 660
   caaactggaa caacactcaa ccctatctcg gtctattctt ttgatttata agggattttg 720
   ccgatttcgg cctattggtt aaaaaatgag ctgatttaac aaaaatttaa cgcgaatttt 780
   aacaaaatat taacgcttac aatttcaggt ggcacttttc ggggaaatgt gcgcggaacc 840
   cctatttgtt tatttttcta aatacattca aatatgtatc cgctcatgag acaataaccc 900
   tgataaatgc ttcaataata ttgaaaaagg aagagtatga gtattcaaca tttccgtgtc 960
   gcccttattc ccttttttgc ggcattttgc cttcctgttt ttgctcaccc agaaacgctg 1020
   gtgaaagtaa aagatgctga agatcagttg ggtgcacgag tgggttacat cgaactggat 1080
   ctcaacagcg gtaagatcct tgagagtttt cgccccgaag aacgttttcc aatgatgagc 1140
   acttttaaag ttctgctatg tggcgcggta ttatcccgta ttgacgccgg gcaagagcaa 1200
   ctcggtcgcc gcatacacta ttctcagaat gacttggttg agtactcacc agtcacagaa 1260
   aagcatctta cggatggcat gacagtaaga gaattatgca gtgctgccat aaccatgagt 1320
   gataacactg cggccaactt acttctgaca acgatcggag gaccgaagga gctaaccgct 1380
   tttttgcaca acatggggga tcatgtaact cgccttgatc gttgggaacc ggagctgaat 1440
   gaagccatac caaacgacga gcgtgacacc acgatgcctg tagcaatggc aacaacgttg 1500
   cgcaaactat taactggcga actacttact ctagcttccc ggcaacaatt gatagactgg 1560
   atggaggcgg ataaagttgc aggaccactt ctgcgctcgg cccttccggc tggctggttt 1620
   attgctgata aatctggagc cggtgagcgt ggctctcgcg gtatcattgc agcactgggg 1680
   ccagatggta agccctcccg tatcgtagtt atctacacga cggggagtca ggcaactatg 1740
   gatgaacgaa atagacagat cgctgagata ggtgcctcac tgattaagca ttggtaggaa 1800
   ttaatgatgt ctcgtttaga taaaagtaaa gtgattaaca gcgcattaga gctgcttaat 1860
   gaggtcggaa tcgaaggttt aacaacccgt aaactcgccc agaagctagg tgtagagcag 1920
   cctacattgt attggcatgt aaaaaataag cgggctttgc tcgacgcctt agccattgag 1980
   atgttagata ggcaccatac tcacttttgc cctttagaag ggaaagctgg gcaagatttt 2040
   ttacgtaata acgctaaaag ttttagatgt gctttactaa gtcatcgcga tggagcaaaa 2100
   gtacatttag gtacacggcc tacagaaaaa cagtatgaaa ctctcgaaaa tcaattagcc 2160
   tttttatgcc aacaaggttt ttcactagag aatgcattat atgcactcag cgcagtgggg   2220
   cattttactt taggttgcgt attggaagat caagagcatc aagtcgctaa agaagaaagg   2280
   gaaacaccta ctactgatag tatgccgcca ttattacgac aagctatcga attatttgat   2340
   caccaaggtg cagagccagc cttcttattc ggccttgaat tgatcatatg cggattagaa   2400
   aaacaactta aatgtgaaag tgggtcttaa aagcagcata acctttttcc gtgatggtaa   2460
   cttcactagt ttaaaaggat ctaggtgaag atcctttttg ataatctcat gaccaaaatc   2520
   ccttaacgtg agttttcgtt ccactgagcg tcagaccccg tagaaaagat caaaggatct   2580
   tcttgagatc ctttttttct gcgcgtaatc tgctgcttgc aaacaaaaaa accaccgcta   2640
   ccagcggtgg tttgtttgcc ggatcaagag ctaccaactc tttttccgaa ggtaactggc   2700
   ttcagcagag cgcagatacc aaatactgtc cttctagtgt agccgtagtt aggccaccac   2760
   ttcaagaact ctgtagcacc gcctacatac ctcgctctgc taatcctgtt accagtggct   2820
   gctgccagtg gcgataagtc gtgtcttacc gggttggact caagacgata gttaccggat   2880
   aaggcgcagc ggtcgggctg aacggggggt tcgtgcacac agcccagctt ggagcgaacg   2940
   acctacaccg aactgagata cctacagcgt gagctatgag aaagcgccac gcttcccgaa   3000
   gggagaaagg cggacaggta tccggtaagc ggcagggtcg gaacaggaga gcgcacgagg   3060
   gagcttccag ggggaaacgc ctggtatctt tatagtcctg tcgggtttcg ccacctctga   3120
   cttgagcgtc gatttttgtg atgctcgtca ggggggcgga gcctatggaa aaacgccagc   3180
   aacgcggcct ttttacggtt cctggccttt tgctggcctt ttgctcacat gacccgaca    3239

## Description

The invention relates to a process for preparing of α-oxyfunctionalized carbonyl compounds from α-ketocarbonyl compounds via enzymatic reduction.

### Background of the Invention

a-oxyfunctionalized carbonyl compounds are indispensable building blocks for asymmetric synthesis due to their versatile functional groups, which are easily transformed to other functionalities, e.g., diols, halo or amino derivatives, and epoxides. Indeed, optically active α-hydroxy acids or ketones have been successfully utilized as starting materials for the asymmetric synthesis of a variety of biologically active molecules (e.g. ephedrines, angiotermin converting enzyme inhibitors, leukotriene antagonists, antifungal azoles, and cerebrosides). Clearly, the convenient and efficient synthesis of optically active α-hydroxy carbonyl compounds is of timely significance and in urgent demand. In essence, the potential of this biocatalytic reaction in synthesizing a library of chiral hydroxy carbonyls is enormous.

In classical organic chemistry the regiospecific reduction of a single keto group from an α-diketone, without reducing the second keto-group, is almost impossible, a stereospecific reduction of one of said keto groups is even more problematic. This applies mutatis mutandis to the regiospecific reduction of the keto group in α-ketoaldehydes and ketoacids.

On the other hand, processes for preparing optically active β-hydroxyketone do exist, these protocols are, however, not suitable for the preparation of α-hydroxyketones. Further, enzymes capable of producing α-hydroxy carbonyl compounds are known in the art (Nakagawa, J. et al., J. Biol. Chem. 277:17883-17891 (2002); Bryn, K. et al., Eur. J. Biochem., 18:116-119 (1971)), these enzymes are not specific and do not only catalyze the reductions of the desired ketogroup but also reduce other functional residues (e.g. the second keto group or aldehyde groups).

Many useful organic compounds, such as pharmaceuticals and food additives, have asymmetric carbon atoms, and enantiomeric forms of these substances exist. In most cases, only one enantiomer is useful as a biologically active substance, whereas the others enantiomer does not show such activity and may even have a harmful effect. Racemic mixtures of such compounds often cannot be used, especially for pharmaceuticals. Therefore, the troublesome resolution of the racemic mixture by means of a conventional organic synthetic process for such optically active substances is unavoidable.

The acetic acid bacterium *Gluconobacter oxydans* is utilized in large scale industrial processes for the stereoselective conversion of organic molecules (e.g. in the vitamin C production). This conversion is catalyzed by proteins, the so-called oxidoreductases. A number of said oxidoreductases were characterized so far (Deppenmeier U. et al.,. Appl. Microbiol. Biotechnol., 60:233-242 (2002)) but none of them react on α-ketocarbonyl compounds.

### Short Description of the Invention

A new class of enzymes was isolated from the acetic acid bacterium *Gluconobacter oxydans* that catalyze a regioselective reduction of α-ketocarbonyl compounds (e.g. α-diketones, α-keto acids and α-ketoaldehydes) to form the respective α-hydroxy carbonyl compounds. In case of α-diketones only the carbonyl moiety adjacent to the shorter alkyl chain of the molecule is reduced. α-ketoaldehydes are exclusively reduced at the keto moiety, thus forming only hydroxyaldehydes. A reduction of the chemically more reactive aldehyde moiety does not occur. This applies mutatis mutandis to α-keto carbonic acids, which are exclusively reduced at the keto moiety. The enzymes selectively produce the respective optically-active isomers, i.e. provide for an enantioselective reduction. All enzymes are dependent on the presence of the cofactor NAD(P)H. A process for regenerating said cofactor was developed, wherein an aldehyde-dehydrogenase converted the NAD(P) back into the reduced form so that the cofactor is only required in catalytic amounts.

These new biocatalysts are innovative tools in the preparation of optically pure key intermediates for pharmaceutical products in current and future industrial processes. The invention thus provides
(1) a process for the enzymatic regio- and enantioselective production of α-hydroxy carbonyl compounds which comprises reacting an α-ketocarbonyl compound with an α-ketocarbonyl reductase derived from Gluconobacter oxydans, wherein the α-ketocarbonyl reductase
   (i) has an apparent molecular weight of 20 to 52 kDa and an activity to selectively reduce the α-keto moiety in α-ketoaldehydes and/or α-ketoesters and/or the keto moiety of a vicinal diketone that carries the shorter alkyl chain of the molecule; and
   (ii) comprises the amino acid sequence of Gox0313 (SEQ ID NO:2), Gox0502 (SEQ ID NO:4), Gox0644 (SEQ ID NO:6), Gox0646 (SEQ ID NO:8) Gox1615 (SEQ ID NO:10), Gox2107 (SEQ ID NO:12), Gox2684 (SEQ ID NO:14), or a mutant thereof having at least 80% sequence identity with the native reductases of SEQ ID NO:2, 4, 6, 8, 10, 12 or 14 and having the enzymatic activity of the respective native reductase of SEQ ID NO:2, 4, 6, 8, 10, 12 or 14;
(2) a preferred embodiment of (1) above, wherein the reducing cofactor NAD(P)H is applied in catalytic amounts and is regenerated with acetaldehyde and an aldehyde dehydrogenase, preferably an aldehyde dehydrogenase derived from *Gluconobacter* oxydans;
(3) a preferred embodiment of (2) above, wherein the aldehyde dehydrogenase derived from Gluconobacter oxydans is Gox1122 (SEQ ID NO:16), or a fragment thereof having the activity of the aldehyde dehydrogenase Gox1122, or a mutant thereof having at least 90% sequence identity with the native aldehyde dehydrogenase of SEQ ID NO:16 and having the activity of the aldehyde dehydrogenase Gox1122.

The biocatalytic process described in the present invention allows an efficient and economic production of the target α-hydroxy carbonyl compounds and replaces multi-step processes of classical organic chemistry. Further advantages of the process are its low energy demand, the use of cheap starting products, a reduced demand for hazardous solvents, and an environmentally positive easy access of the reagents (enzymes) from natural sources (microorganisms). Finally, the process provides for the production of α-hydroxy carbonyl compounds, which are quite difficult to be produced by hitherto known classical organic synthesis methods, or are impossible to be produced by such methods.

### Short Description of the Figures

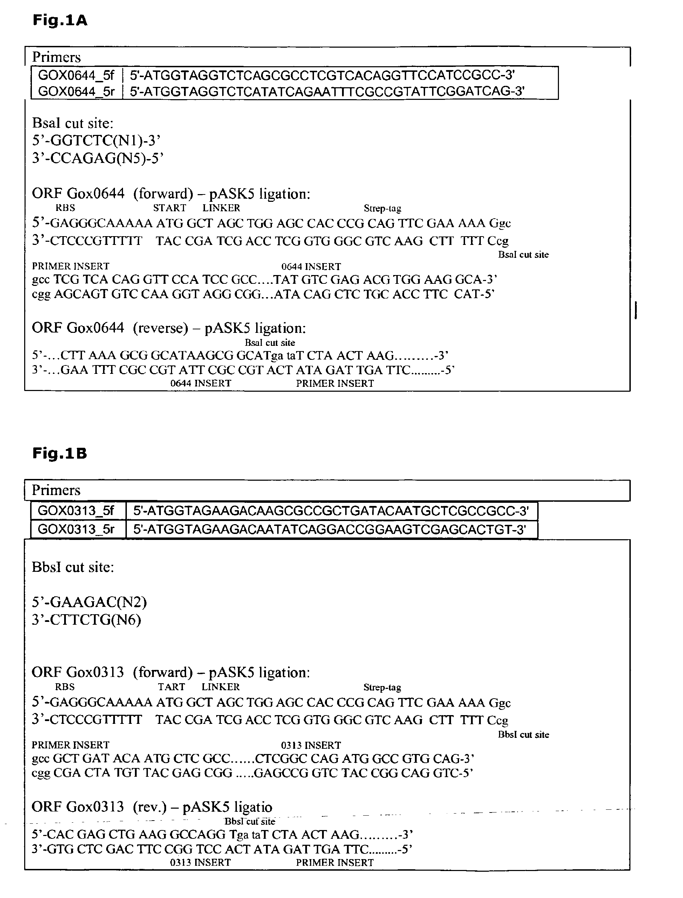

Fig.1: Cloning strategies using BsaI (A) or using BbsI (B).

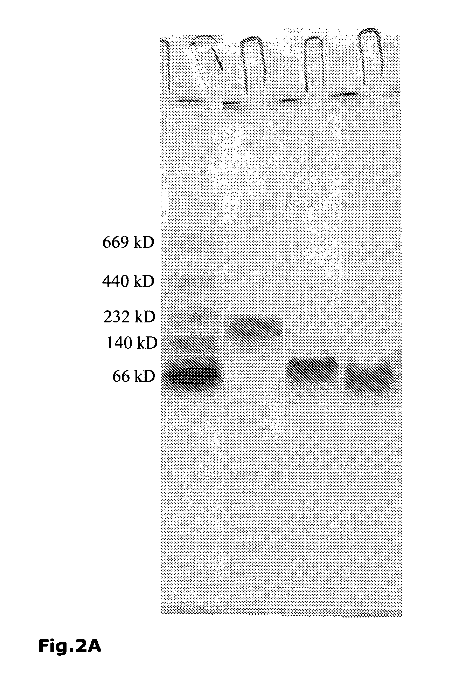

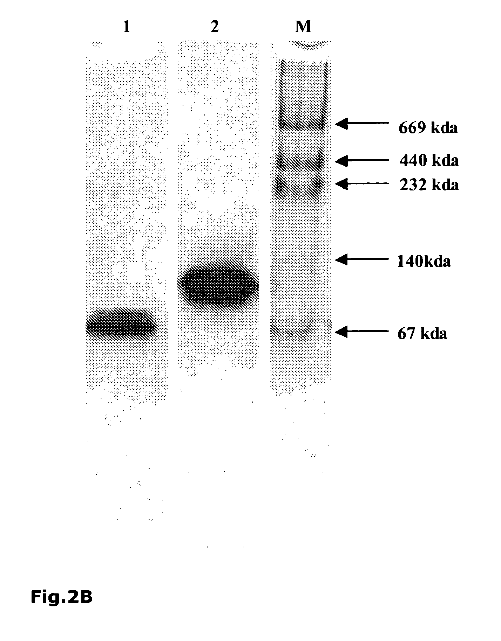

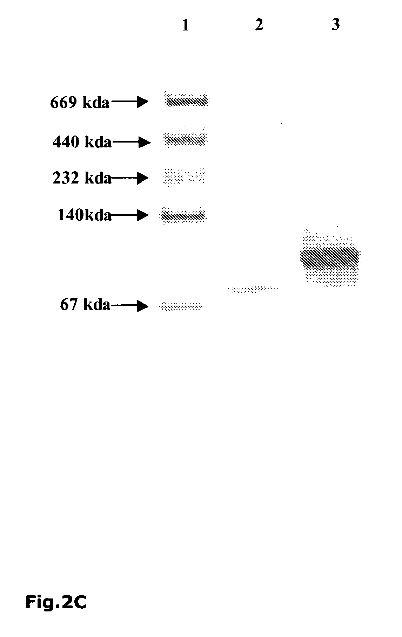

Fig. 2: (A) NATIVE-PAGE analysis of Gox0313 Gox0644 and Gox1615, lane 1 represents the molecular weight standards of 66 kDa, 140 kDa, 232 kDa, 440 kDa and 669 kDa, lanes 2, 3 and 4 represent Gox0313, Gox1615 and Gox0644, respectively. (B) NATIVE-PAGE analysis of Gox2107 and Gox0502, arrows indicate molecular weight standards [form the top: thyroglobulin (669 kDa), ferritin (440 kDa), catalase (232 kDa), lactate dehydrogenase (140 kDa), bovine serum albumin (67 kDa)], M: Molecular weight marker, lane 1: Gox2107 (2 µg protein), lane 2: Gox0502 (2 µg protein). (C) NATIVE-PAGE analysis of Gox2684 and Gox0646, arrows indicate molecular weight standards [form the top: thyroglobulin (669 kDa), ferritin (440 kDa), catalase (232 kDa), lactate dehydrogenase (140 kDa), bovine serum albumin (67 kDa)]. M: Molecular weight marker, lane 2: Gox2684 (1 µg protein); Lane 3: Gox0646 (2 µg protein).

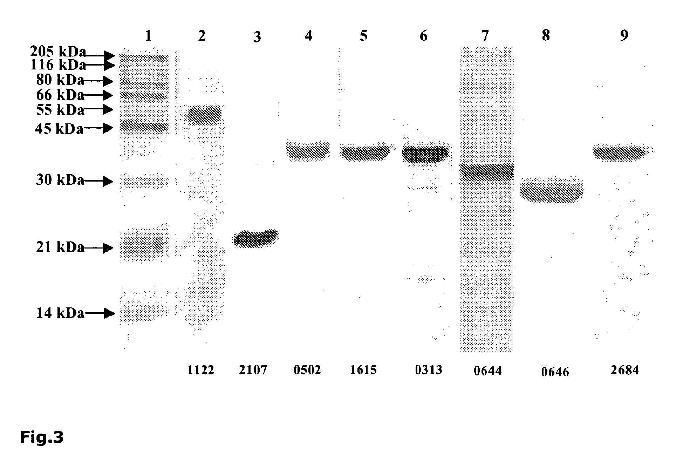

Fig. 3: SDS-PAGE analysis of Gox1122, Gox2107, Gox0502, Gox1615, Gox0313 Gox0644, Gox0646and Gox2684. The proteins were compared to molecular weight marker to determine approximate size (14 kDa, 21 kDa, 30 kDa, 45 kDa, 55 kDa, 66 kDa, 80 kDa, 97 kDa, 116 kDa and 205 kDa molecular weight markers lane 1). Gox 1122 (line 2) = 50.5 kDa, Gox 2107 (lane 3) = 21.7 kDa, Gox0502 (lane 4) = 39.1 kDa, Gox1615 (lane 5) = 37.1 kDa, Gox0313 (lane 6) = 36.3 kDa, Gox0644 (lane 7) = 31.5 kDa, Gox0646 (lane 8) = 27.7 kDa, and Gox2684 (lane 9) = 39.4 kDa.

### Detailed Description of the invention

Genome sequencing *Gluconobacter oxydans* surprisingly revealed various hitherto unknown oxidoreductases. Eight such enzymes are preferred embodiments of this invention and are particularly suitable for the stereo- and regioselective production of α-hydroxycarbonyl compounds from α-ketocarbonyl compounds.
The process for the enzymatic regio- and enantioselective production of α-hydroxy carbonyl compounds of aspect (1) of the invention comprises reacting an α-ketocarbonyl compound with an α-ketocarbonyl reductase derived from *Gluconobacter oxydans* that
(i) has an apparent molecular weight of 20 to 52 kDa and an activity to selectively reduce the α-keto moiety in α-ketoaldehydes and/or α-ketoesters or, with respect to α-diketones, the keto group which is proximal to the shorter chain of the molecule, and
(ii) comprisies the amino acid sequence of the following α-ketocarbonyl reductases: Gox0313 (SEQ ID NO:2), Gox0502 (SEQ ID NO:4), Gox0644 (SEQ ID NO:6), Gox0646 (seq ID NO:8), Gox1615 (SEQ ID NO:10), Gox2107 (SEQ ID NO:12), Gox2684 (SEQ ID NO:14), and mutants thereof as defined above. These reductases have the following physico-chemical properties:
   Gox0313 belongs to the class of Zinc-binding dehydrogenases and is composed of five identical subunits with molecular masses of 36.5 kDa. The enzymes catalyses the NADH-dependent reduction of a variety of α-diketones, α-ketoaldehydes, and other aliphatic/aromatic aldehydes, forming α-hydroxyketones, α-hydroxyaldehydes, and alcohols, respectively. The enzymes also uses NADPH but activities are lower. Substrates showing the highest activities were 2,3 pentanedione (Km = 3.1 mM, Vmax = 97 U/mg protein), 2,3 hexanedione and acetaldehyde (Km = 0.5 mM, Vmax = 95 U/mg protein). The optimal pH value is 7 and the optimal temperature for biotransformation was 30 °C.
      In addition, the enzyme was also able to catalyze the NAD-dependent oxidation of some primary and secondary alcohols.
   Gox0502 belongs to the class of NADH:flavin oxidoreductases and is composed of two identical subunits with molecular masses of 39.2 kDa. The enzymes catalyses the NADPH-dependent reduction of a variety of α-ketoaldehydes, α-diketones, short chain vinyl ketones and quinones, forming α-hydroxyaldehydes, α-hydroxyketones and ketones. Substrates showing the highest activities are 3-butene-2-one (Km = 0.1 mM, Vmax = 21 U/mg protein), and phenylglyoxal. The optimal pH value is 7 and the optimal temperature for biotransformation is 30 °C. The protein contains 1 mol of flavinmononucleotide (FMN) per mol of subunit.
   Gox0644 belongs to the class of aldo-keto reductases and is composed of two identical subunits with molecular masses of 31.7 kDa. The enzymes catalyse the NADPH-dependent reduction of a variety of α-diketones and α-ketoaldehydes forming α-hydroxyketones and α-hydroxyaldehydes. Substrates showing the highest activities are 2,3 pentanedione (Km = 5.6 mM, Vmax = 79 U/mg protein), phenylglyoxal (Km = 4.7 mM, Vmax = 93 U/mg protein), and phenyl-1,2-propanedione. The optimal pH value is 6.8 and the optimal temperature for biotransformation is 37 °C.
   Gox0646 belongs to the class of short chain dehydrogenase/reductase family and is composed of two identical subunits with molecular masses of 27.7 kDa. The enzymes catalyse the NADPH-dependent reduction of a variety of α-diketones forming α-hydroxyketones. Substrates showing the highest activities are 2,3 pentanedione (Km = 9 mM, Vmax = 10 U/mg protein) and 2,3-hexanedione. The optimal pH value is 7 and the optimal temperature for biotransformation is 32 °C. Gox1615 belongs to the class of aldo-keto reductases and is composed of two identical subunits with molecular masses of 37.2 kDa. The enzymes catalyse the NADPH-dependent reduction of a variety of α-diketones and α-ketoaldehydes forming α-hydroxyketones and α-hydroxyaldehydes. Substrates showing the highest activities are 2,3 pentanedione, 2,3-hexanedione (Km = 3.2 mM, Vmax = 6 U/mg protein), phenylglyoxal, and methylglyoxal (Km = 3.8 mM, Vmax = 22 U/mg protein). The optimal pH value is 6.5 and the optimal temperature for biotransformation is 30 °C.
   Gox2107 belongs to the class of flavodoxin proteins and is composed of three identical subunits with molecular masses of 21.9 kDa. The enzymes catalyses the NADPH-dependent reduction of the α-ketoaldehyde phenylglyoxal and a variety of short chain vinyl ketones, and quinones forming 2-hydroxy-2-phenylacetaldehyde, ketones and reduced quinones respectively. Substrates showing the highest activities are 1,4-naphtoquinone (Km = 0.01 mM, Vmax = 217 U/mg protein), 3-buten-2-one (Km = 3.8 mM, Vmax = 119 U/mg protein) and phenylglyoxal. The optimal pH value is 6 and the optimal temperature for biotransformation is 35 °C. The protein contains 1 mol of flavinadenin-dinucleotide (FAD) per mol of subunit.
   Gox2684 belongs to the class of NADH:flavin oxidoreductases and contains a FMN binding domain. The enzyme is composed of two identical subunits with molecular masses of 39.4 kDa. The biocatalyst is able to reduce a variety of α-ketoaldehydes, α-diketones and quinones. Substrates with highest activities were 2,3 hexanedione (2.3 U/mg protein) and phenylglyoxal (2.1 U/mg protein).

The modifications, i.e. mutants of the aforementioned native enzymes of SEQ ID NOs: 2, 4, 6, 8, 10, 12 and 14 include addition, substitution, inversion and deletion mutants, having at least 80%, preferably at least 90%, most preferably at least 95% sequence identity with the native enzyme on the amino acid basis (notably mutants, wherein 1 to 20, preferably 1 to 10, consecutive or separated amino acid residues are added, substituted, inverted and/or deleted; for substitution mutants conservative substitution is particularly preferred), provided, however, that said modified enzymes have the enzymatic activity of the native enzymes.

For Gox0644, it is particularly preferred that the modifications are in the following regions (basis numbering shown in SEQ ID NO:6): region 1: 1-10 (N-terminus); region 2: 66-72; region 3: 112-120 (loop between β4 and α4); region 4: 192-202 (loop between β7 and H1); region 5: 266-279 (loop at C-terminus); particularly amino acids K234, R240, W30, A272.

For Gox1615 it is particularly preferred that the modifications are in the following regions (basis numbering of SEQ ID NO: 10): region 1: 1-10 (N-terminus); region 2: 321-332 (loop at C-terminus); region 3: 84-90 and region 4: 233-252.

For Gox0313, Gox0646, Gox2107, Gox0502, Gox2684, Gox1122 it is preferred that the modifications are in the N-terminal and C-terminal regions

In the process aspect (1) it is preferred that the reaction is conducted in cell free environment, wherein the α-ketocarbonyl reductase is present as free enzyme or is presented as a cell lysate or as immobilized enzyme. Alternatively the reaction may be conducted in the presence of transformants that produce the α-ketocarbonyl reductase *in-situ*.

The α-ketocarbonyl compound that may be reduced is selected from α-diketones, α-keto acids, α-keto esters, α-ketoaldehydes and vinylogous/phenylogous compounds thereof (i.e. 4-oxo-alk-2,3-en-aldehydes and acetophenone-4-carbocylic acid). Other α-ketocarbonyl compound that may be reduced is selected from α-keto acid chlorides and/or α-keto acid anhydrides.

The reaction may be performed in the batch mode or the continuous mode.

It is particularly preferred in the process according aspect (1) that
the α-ketocarbonyl reductase is Gox0313 (SEQ ID NO:2) or a mutant thereof having at least 80% sequence identity with the native reductases of SEQ ID NO:2 and having the enzymatic activity of the native reductase of SEQ ID NO:2, and the ketocarbonyl compound to be reduced is an α-diketone or an α-ketoaldehyde; or
the α-ketocarbonyl reductase is Gox0502 (SEQ ID NO:4) or a mutant thereof having at least 80% sequence identity with the native reductases of SEQ ID NO:4 and having the enzymatic activity of the native reductase of SEQ ID NO:4, and the ketocarbonyl compound to be reduced is an α-diketone or an α-ketoaldehyde; or
the α-ketocarbonyl reductase is Gox0644 (SEQ ID NO:6) or a mutant thereof having at least 80% sequence identity with the native reductases of SEQ ID NO:6 and having the enzymatic activity of the native reductase of SEQ ID NO:6, and the ketocarbonyl compound to be reduced is an α-diketone, an α-keto ester or an α-ketoaldehyde; or
the α-ketocarbonyl reductase is Gox0646 (SEQ ID NO:8) or a mutant thereof having at least 80% sequence identity with the native reductases of SEQ ID NO:8 and having the enzymatic activity of the native reductase of SEQ ID NO:8, and the ketocarbonyl compound to be reduced is an α-diketone, or
the α-ketocarbonyl reductase is Gox1615 (SEQ ID NO:10) or a mutant thereof having at least 80% sequence identity with the native reductases of SEQ ID NO:10 and having the enzymatic activity of the native reductase of SEQ ID NO:10, and the ketocarbonyl compound to be reduced is an α-ketoaldehyde or an α-diketone; or
the α-ketocarbonyl reductase is Gox2107 (SEQ ID NO:12) or a mutant thereof having at least 80% sequence identity with the native reductases of SEQ ID NO:12 and having the enzymatic activity of the native reductase of SEQ ID NO:12, and the ketocarbonyl compound to be reduced is an α-ketoaldehyde;or
the α-ketocarbonyl reductase is Gox2684 (SEQ ID NO:14) or a mutant thereof having at least 80% sequence identity with the native reductases of SEQ ID NO:14 and having the enzymatic activity of the native reductase of SEQ ID NO:14, and the ketocarbonyl compound to be reduced is an α-diketone, or an α-ketoaldehyde. According to aspects (2) and (3) of the invention the reducing cofactor NADPH is applied in catalytic amounts and is regenerated with acetaldehyde and an aldehyde dehydrogenase. Such aldehyde dehydrogenase is preferably derived from *Gluconobacter oxydans,* and in particular is Gox1122 (SEQ ID NO:16) or a fragment thereof having the activity of the aldehyde dehydrogenase Gox1122, or a mutant thereof having at least 90% sequence identity with the native aldehyde dehydrogenase of SEQ ID NO:16 and having the activity of the aldehyde dehydrogenase Gox1122.

Gox1122 is a NADP-dependent aldehyde dehydrogenase and exhibited an apparent molecular mass of 50.1 kDa. The subunit mass was 50.5 kDa, indicating a monomeric structure of the native enzyme. The *Km* value of Gox1122 for acetaldehyde, estimated by using several concentrations of the aldehyde from 0.0025 to 10 mM, was 255 µM. The apparent *Km* value for NADP was 73 µM. The apparent Vmax value, estimated at a fixed 0.5 mM NADP concentration with acetaldehyde as the varied substrate, was 196 U/mg protein. The enzyme also oxidized other short-chained aliphatic and aromatic aldehydes with lower rates. The optimal pH value is 8 and the optimal temperature for biotransformation was 30 °C. No reverse reduction reaction of acetic acid was observed using NADPH a pH range of 4-10. The enzyme is used in a combined enzymatic reaction between the NADPH-dependent reductases (see above) that form α-hydroxy carbonyl compounds. In this combined reaction Gox1122 catalyzes the conversion of acetaldehyde into acetic acid and of NADP into NADPH.

As to the mutations of the aldehyde dehydrogenase it is referred to the mutations discussed in connection with the reductases above.

The reaction according to aspect (2) and (3) is further explained in the following reaction scheme 1.

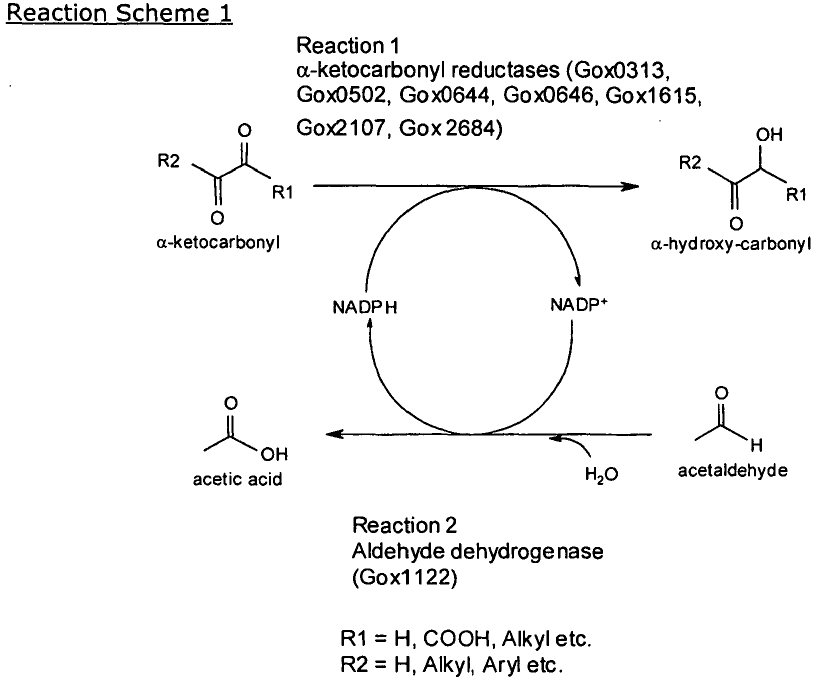

The α-ketocarbonyl reductases used in aspect (1) of the invention are α-ketocarbonyl reductases comprising the amino acid sequence Gox0502 (SEQ ID NO:4), Gox0644 (SEQ ID NO:6), Gox0646 (SEQ ID NO:8), Gox1615 (SEQ ID NO:10), Gox2107 (SEQ ID NO:12) and Gox2684 (SEQ ID NO:14) and mutants thereof as defined above. Particular preferred α-ketocarbonyl reductases are that having the sequence of SEQ ID NO: 4, 6, 8, 10, 12 or 14.

In this context it is to be noted that Blast searches at NCBI in the databases "Patented protein sequences" and "Non-redundant protein sequences" using the algorithm "blastp" and the matrix "BLOSUM62" were performed for SEQ ID NOs:2, 4, 6, 8, 10, 12, 14 and 16. There were no homologous proteins (cut off = 75% identity) found for SEQ NO 4, 6, 10, 12, 14 and 16 in said databases. SEQ ID NO: 2 was 100% identical to Sequence 4 from US patent 6,242,228, which describes xylulose reduction to xylitol using the protein from sequence 4. Furthermore, SEQ ID NO: 2 revealed 81% identity to a protein from *Gluconacetobacter diazotrophicus* PAI 5 which has not been characterized yet and has no known activity.

SEQ ID NO:8 showed 99% identity to the acetate adaptation protein AapL from *Acetobacter aceti*. It was found that AapL is involved in the adaptation towards high acetate concentrations in the growth medium. A specific physiological function or an enzyme activity was not described (Steiner and Sauer, Appl. Environ. Microbiol. 67:5474-81 (2001))

An enzyme having the first 9 N-terminal amino acid residues of Gox0502 (SEQ ID NO:4) is disclosed in Shinagawa et al., Biosci. Biotechnol. Biochem. 72:260-4 (2008). Said enzyme is however not found in the Blast searches set forth above.

Further disclosed are DNA sequences encoding the above mentioned α-ketocarbonyl reductases or mutants thereof. It is particularly preferred that said DNA sequences have the sequence shown in SEQ ID NO:1, 3, 5, 7, 9, 11 or 13. Homologues of said DNA sequences with an identity of greater than 80% on the nucleotide basis are also included.

Also disclosed are vectors or expression vectors comprising at least one of the above mentioned DNA sequences. Expression vectors strongly depend on the type of the expression host and include that of the pASK-IBA series for the expression in *E. coli* and that of the pGEX series (Schleyer et al., 2008, Int. J. Food Microbiol. In press) for (over)expression in *Gluconobacter* species.

Also disclosed are host cells containing at least one of the above mentioned DNA sequences and/or the above mentioned expression vectors. Any type of prokaryotic host cell is applicable according to the present invention, Gluconobacter species and *E.coli* are, however, particularly preferred.

Furthermore disclosed is a process for preparing an α-ketocarbonyl reductase derived from *Gluconobacter oxydans* as utilized of the method of (1) above, which comprises culturing an above mentioned host cell and isolating the α-ketocarbonyl reductase from the culture broth or the host cells. The process may further contain suitable purification steps.

The invention is described in more detail in the following Example.

### Examples

### Materials and Methods

Microorganisms and culture conditions: *G. oxydans* 621H (DSMZ 2343) was cultivated in a complex medium containing 0.6 % (w/v) yeast extract and 2 % (w/v) mannitol at 30 °C. *E. coli* strains were cultivated in Luria broth (LB) (10 g trypton, 5 g yeast extract, 10 g NaCl per liter) at 37 °C. For enzyme overproduction *E. coli* DH5α was grown on modified Maximal Induction (MI) medium (Mott, J.E. et al., Proc. Natl. Acad. Sci. USA 82:88-92 (1985)) containing 3.2 % (w/v) tryptone, 2 % (w/v) yeast extract with additions of M9 salts (1 mM CaCl₂, 1 mM MgSO₄, 1 µM FeNH₄ citrate). Ampicillin was added to a final concentration of 100 µg/ml.

Preparation and manipulations of nucleic acids: The plasmid used for cloning and for expression of *gox0313, gox0502, gox0644, gox0646, gox1615, gox2107, gox2884 and gox1122* in *E. coli* was pASK-IBA5 (IBA GmbH, Goettingen, Germany; SEQ ID NO:33). The multiple cloning site within the vector contained restriction sites for *Bsa*I which allowed the precise fusion of the structural gene with a N-terminal *Strep*-tag^{®} II. The vector also encoded for ampicillin resistance and had a controllable *tet* A promoter/repressor system.

The pASK-IBA5 expression vector (SEQ ID NO:33) contains a strong regulatable Tet promoter, start and stop codon, ribosomal binding site, the Strep TagII sequence, linker sequence to cleave the Strep TagII and restriction endonuclease sites within the multiple cloning sequence (MCS). Furthermore, the vector has been optimized for protein expression in *E. coli* strains, generating large amounts of homologous recombinant protein.

Plasmid DNA was prepared from *E. coli* with GeneJET Plasmid Miniprep Kits (Fermentas GmbH). An *E. coli*-culture (3 ml) was precultured overnight in LB-medium containing the respective antibiotic at 37 °C, was transferred to sterile Eppendorf-Cups and centrifuged at 12,000 rpm for 5 min (Biofuge 15, Heraeus GmbH, Osterode, Germany). The cell pellet was taken up in 250 µl resuspension solution (Fermentas GmbH), 250 µl lysis solution (Fermentas GmbH) was added and shaked until a clear solution was obtained. The solution was neutralized by addition of 350 µl neutralization solution (Fermentas GmbH) and shaking. After centrifugation at 12,000 rpm for 5 min, the supernatant was transferred to the GeneJET mini-column, which was subsequently centrifuged at 12,000 rpm for 30s). The primary eluent was discarded. The column was then treated twice with 500 µl wash solution (Fermentas GmbH), wherein the solution was applied to the column and centrifuged at 12,000 rpm for 30 s. The eluent was again discarded. The plasmid-DNA was eluted from the column by applying 50 µl elution-buffer (Fermentas) at pH 8,0. After incubation for 2 min the column was centrifuged (12000 rpm, 1 min) and the eluated DNA was stored at 4 °C.

DNA Isolation: Total DNA from *G. oxydans* was isolated by the CTAB method (Ausubel, F.M. in Ausubel, Brent, Kingston, Moore, Seidman and Struhl (eds): Current Protocols in Molecular Biology, John Wiley & Sons, Inc., New York, Vol 5: pp 2-11 (2002)). *G. oxydans* was precultured in 50 ml yeast-mannitol-medium up to the stationary phase. The cells were sedimented by centrifugation (8,000 rpm, 4 °C, 15 min im SS 34-Rotor in the Sorvall centrifuge RC-5B (Fa. Du Pont, Bad Homburg) and the pellet was resuspended in 0,95 ml TE-buffer (Tris/HCl 10 mM and EDTA 1 mM, pH 8.0). After addition of 50 µl 10 % (w/v) SDS und 5 µl proteinase K solution (20 mg/ml) the mixture was incubated at 37 °C for 1 h. Thereafter 0,18 ml 5 M NaCl und 0,15 ml CTAB/NaCl solution (700 mM NaCl and 275 mM CTAB) was added, mixed and the suspension was kept in a water-bath at 65 °C for 2 h. For extraction of the proteins 0,133 ml (1 vol.) phenol/CHCl₃ 24:1 (v/v) was added and carefully mixed. After centrifugation the upper aqueous phase was extracted with 0.5 vol. CHCl₃/isoamylalcohol 1:1 (v/v). For precipitation of DNA 0.6 vol. isopropanol was added to the resulting aqueous phase followed by centrifuging at 6,000 rpm, 4 °C for 15 min. The pellet was washed with 0,1 ml cold 70 % (v/v) aqueous Ethanol, was dried for 10 min at 60 °C and subsequently taken up in 0,2 ml TE-buffer with vortexing. Thereafter the DNA was incubated for 10 min on a water-bath at 70° C, centrifuged and was pipetted to obtain a homogenous viscous solution. The DNA was stored at 4 °C.

Polymerase chain reaction (PCR), Primer Construction: Primer for PCR were constructed using the program Primer D'Signer 1.1 (IBA GmbH, Goettingen, Germany). The oxidoreductase genes were amplified by PCR with the introduction of BsaI, BbsI or BsmBI cut sites to complement the digested plasmid vector. (see below). Alternatively, *BsmBI* or *BbsI* could be introduced at the ends of the PCR fragments when the genes contained internal *BsaI* sites. Therefore, the primers for all the soluble dehydrogenase/oxidoreductase genes were constructed with either, *BsaI, BsmBI* or *BbsI* restriction endonuclease cut sites introduced at both ends of the gene (see Table 1 below).

The primers with *Bsa*I restriction sites were: *gox0644, gox0502, gox2107* and *gox1122*. However, in the -case where the gene contained internal cut sites with *BsaI* the alternate restriction enzymes *BsmBI* or *BbsI* were used for the primer construction. The alternate enzymes *BsmBI* and *BbsI* are also ideally suited for the use with the pASK-IBA5 vector. The primers *gox0313* contained the *Bbs*I site whereas *gox1615* contained the *BsmBI* site.

**Table 1: Primers used for PCR**

| Primer (SQ ID NO) | Primer sequence | Restriction site introduced |
|---|---|---|
| GOX0313_5f (17) | ATGGTAGAAGACAAGCGCCGCTGATACAATGCTCGCCGCC | *BbsI* |
| GOX0313_5r (18) | ATGGTAGAAGACAATATCAGGACCGGAAGTCGAGCACTGT | *BbsI* |
| GOX0502_5f (19) | ATGGTAGGTCTCAGCGCCCCAACCCTGTTCGATCCCATTG | *BsaI* |
| GOX0502_5r (20) | ATGGTAGGTCTCATATCAGTTGGGGCCGGAGGTGGCG | *BsaI* |
| GOX0644_5f (21) | ATGGTAGGTCTCAGCGCCTCGTCACAGGTTCCATCCGCC | *BsaI* |
| GOX0644_5 (22) | ATGGTAGGTCTCATATCAGAATTTCGCCGTATTCGGATCAG | *BsaI* |
| GOX0646_5f (23) | ATGGTAGGTCTCAAATGCTGAACCTCGATCTGAGCGGC | *BsaI* |
| GOX0646_5r (24) | ATGGTAGGTCTCAGCGCTGATGGTATCGACGGTGCCGCC | *BsaI* |
| GOX1615_5f (25) | ATGGTACGTCTCAGCGCCGCATCCGACACCATCCGCATC | *BsmBI* |
| GOX1615_5r (26) | ATGGTACGTCTCATATCAGTCCCGTGCCGGGGGCGC | *BsmBI* |
| GOX2107_5f (27) | ATGGTAGGTCTCAGCGCCACGAAAATTCTTCTCCTTAACGGC | *BsaI* |
| GOX2107_5r (28) | ATGGTAGGTCTCATATCAGGACGCCGTACCGATGACGTC | *BsaI* |
| GOX2684_5f (29) | ATGGTAGGTCTCAGCGCCACCAGCCTGTTTGAGCCGATTG | *BsaI* |
| GOX2684_5r (30) | ATGGTAGGTCTCATATCAGCGAGCAAGCGGATAATCCGTA | *BsaI* |
| GOX1122_5f (31) | ATGGTAGGTCTCAGCGCCGCTTACGCTACGATCAACCCTTA | *BsaI* |
| GOX1122_5r (32) | ATGGTAGGTCTCATATCAGAACGGCGCGTCGATATCAACA | *BsaI* |

PCR conditions: The amplification reaction mixtures contained 100 ng of total DNA of *G. oxydans*, 0.5 pmol of each oligonucleotide primer, 200 µM each deoxynucleotide triphosphate (dNTP), 5 µl of 10X *Pfu* cloned polymerase buffer, and 2.5 U of *Pfu* (Stratagene, La Jolla, CA, USA) in a total volume of 50 µl. The mixture was subjected to PCR amplification in a DNA thermal cycler (Bio-Rad MyCycler) for 30 cycles. Each cycle was defined by a 45-second denaturing step at 95 °C, a 1-minute annealing step at 55 °C, and a 2-minute 30-second polymerization step at 72 °C. PCR products were analyzed by electrophoretic separation in a 0.8 % agarose gel. PCR fragments were microdialyzed with 0.025 µm membrane filters for 1 h. The size of the PCR fragments was analyzed by electrophoretic separation in a 0.8 % agarose gel and a Tris-acetate buffer system. Clone Development: After generation of PCR products the DNA fragments were cloned into the pASK vector. *Bsa*I, *BsmBI* and *Bbs*I belong to the Type IIS restriction enzymes which cleave the DNA outside their recognition sites. Thereby, the digestion with one single enzyme can generate two different independent sticky ends with 4-base 5'-overhangs allowing unidirectional cloning. Each of these three restriction endonuclease recognize a different cut sequence, but all produce the same 5' to 3' overhang consisting of GCGC that precisely complements the 3' to 5' pASK-IBA5 overhang of CGCG. At the 3' end of the gene the restriction enzymes produce the sequence TATC, which is complementary to the 3' to 5' sequence ATAG on the plasmid.

Vector pASK-IBA5 was digested with *BsaI* for cloning of restriction endonuclease treated PCR products representing genes *gox*0313, *gox*0502, *gox*0644, *gox*0646 *gox*1615, *gox*2107, *gox*2684, and *gox*1122. The cloning strategy is shown in Fig. 1. Fig. 1A describes the sequence complementation of *gox*0644 after restriction digestion with *BsaI.* gox0644 represents all genes using *BsaI* cut sites producing the 5' CGCG 3' sequence and 5' TATCA 3' on the reverse strand, which allows for precise ligation into the pASK vector. Fig. 1B describes the sequence complementation of gox0313 after restriction digestion with *BbsI. gox*0313 represents all genes using *BbsI* cut sites producing the 5' CGCG 3' sequence and 5' TATCA 3' on the reverse strand, which allows for precise ligation into the pASK vector.

Successful incorporation of the PCR oxidoreductase gene construct into the pASK IBA vector was achieved according to the ligation protocol using T4 ligase (New England Biolabs, Ipswich, MA, USA). For this procedure 2 µl T4 ligase buffer was mixed with 2 µl BsaI digested pASK5, 15 µl BsaI (or BbsI or BsmBI as indicated in Tab. 1) digested PCR product and 1 µl of T4 ligase. The mixture was incubated for 1 hour at room temperature. This procedure incorporates the restriction digested gene of interest with a 5' overhang sequence GCGC to the pASK-IBA5 vector with the complementary sequence overhang. The 3' overhang of the gene containing the GATA sequence was fused to the complementary overhanging sequence (Fig.1A-B Schemes of cloning strategies). This protocol achieved a high success rate of ligation based on the high ratio of restriction endonuclease (*BsaI, BbsI, BsmBI*) cut purified PCR product to *BsaI* cut plasmid.

Once ligated the plasmids containing the inserts were transformed into competent *E. coli* DH5 α cells (Inoue H. et al., Gene 96: 23-28 (1990)). Competent *E. coli* cells containing the plasmid with the recombinant oxidoreductase - genes were allowed to outgrow in 500 µl of LB media for one hour at 37°C. Incremental aliquots of 20 µl, 50 µl and 150 µl of *E. coli* DH5α cells were plated on LB plates containing 1 µg/ml ampicillin and incubated at 37°C to insure growth with adequate separation between colonies for easy colony selection.

Colonies were randomly chosen and grown overnight at 37°C in LB broth containing 1ug/ml of ampicillin in order to validate the correct incorporation of the gene of interest in the pASK plasmid. Plasmids were purified as described above. In order to check all aspects associated with the plasmid construct the following plasmid components: Strep TagII, ribosome binding site, linker sequence, start codon, termination codon and a gene insert ligated in the proper reading frame with the start codon, were analyzed by sequencing the corresponding region of the plasmid at the Madison Biotechnology Sequencing center.

Overexpression: Overnight starter cultures of the validated stock pASK-IBA5 oxidoreductases in *E. coli* DH5α were grown at 37°C in 20ml LB medium containing 20 ug of Ampicillin. Culture flasks with 80% headspace provided optimal aeration at shaker speeds of 200 rpm or greater, for the overexpression of heterologous proteins. These starter cultures of currently replicating cells were used to generate rapid cell growth at 37°C when added to yeast extract and tryptone containing Maximal Induction media (MI media in methods). Ampicillin (1 µg/µl) was again added to inhibit ampicillin sensitive organisms. *E. coli* growth and protein expression was optimized on MI media through a biphasic production system. The first phase of growth supported cellular replication, in which heterologous protein production was inhibited at the tetracycline promoter (tet promoter) by the tetracycline repressor protein. The second phase focused on the optimized expression of heterologous protein in which available resources were predominantly directed toward protein synthesis. Protein synthesis was initiated by the addition 100 µl of 2 mg/ml anhydrotetracyclin stock solution for 1 l of cell culture when the culture density reached an OD₆₀₀ of 0.4-0.6. The cells were allowed to grow for an additional four hours to complete protein production

Cells were harvested by centrifugation at 4500 x g at 4 °C. Cell pellets were resuspended in 15 ml Buffer W (100 mM Tris/HCl pH 8.0, 150 mM NaCl) at 4 °C. Protease cocktail set III (10 µl/l cells) (Merck KGaA, Darmstadt, Germany) and DNase I (20 U/I cells) (Abgene, Epsom, UK) were added to the resuspended cell pellets and allowed to incubate on ice for ten minutes. Resuspended cells were lysed by sonication at 4 °C (30-35W for 10 s intervals over 10 min). Lysates were centrifuged at 24,000 x g at 4 °C and the cleared lysate was collected.

At this point the overexpressed soluble oxidoreductase was suspended in the buffer along with other soluble cellular proteins necessitating further purification via the Strep Tag II purification system. Column chromatographies were carried out at 4 °C with a Strep-Tactin Superflow^{®} affinity column (IBA GmbH, Goettingen, Germany) connected to a ÄXTA-FPLC system (Amersham Bioscienes, Piscataway, NJ, USA). A bed volume of 10 ml was equilibrated with two column volumes of Buffer W. Afterwards the clear cell lysate containing the Strep Tag II fused soluble oxidoreductases was applied and the column was washed with five column volumes of Buffer W. To elute the bound protein buffer E (buffer W containing 2.5 mM desthiobiotin) was applied to the column. After collection of the pure biocatalysts the affinity column was regenerated with buffer R (buffer W containing 1 mM HABA).

### Characterization of the Isolated Enzymes:

*Polyacrylamide gel electrophoresis (PAGE):* SDS-PAGE was done on a 14 % (w/v) slab gel as described by Laemmli [Laemmli, 1970] with 4 % (w/v) polyacrylamide stacking gel. Before application samples were diluted 1:1 (v/v) in sample loading buffer (2 % [w/v] SDS, 5 % [v/v] β-mecaptoethanol, 50 % [v/v] glycerin, 20 % [v/v] collecting buffer pH 6.8, 0.001 % [w/v] bromophenol blue) and boiled for 10 min. The following reference proteins (Amersham Bioscienes, Piscataway, NJ, USA) with the indicated molecular masses were used for the measurement of the molecular mass of cytosolic oxidoreductases: myosin (205 kDa), β-galactosidase (116 kDa), phosphorylase b (97 kDa), transferrin (80 kDa), bovine serum albumin (66 kDa), glutamate dehydrogenase (55 kDa), ovalbumin (45 kDa), carbonic anhydrase (30 kDa), and lysozyme (14 kDa).

*Molecular Sieve Chromatography:* A Sephacryl 300HR 16/60 chromatography (Amersham) with reference proteins Blue Dextran (2 MDa), Thyroglobulin (669 kDa), Ferritin (440 kDa), Catalase (232 kDa), Aldolase (140 kDa) and Olvabumin (43 kDa) were used to determine the native weight of the aldehyde dehydrogenases. The native molecular mass for each protein was determined by comparison to the standard curve generated by the molecular weight standards which were applied to a gel filtration column. The distances each molecular weight standard traveled (Rf) was plotted against the logarithm of the molecular mass of the standards.

*Determination of pH and temperature optima and enzyme kinetics:* The optimum pH for enzyme activities was determined by a modified method of Salusjärvi, T. et al., Appl. Microbiol. Biotechnol. 65:306-314 (2004) using the following buffer system (50 mM final concentration each): sodium acetate pH 4.5 - 6.5, potassium phosphate pH 6.5 - 8.0, Tris-HCl pH 8.0 - 10. Temperature optima for the biocatalysts were determined under standard reaction conditions at temperatures ranging from 20 - 80 °C. The Michaelis constant (Km) for the proteins was determined from purified enzyme by Lineweaver-Burk plots. Assays were performed at optimum pH for each enzyme and at 30 °C using the top substrate and a targeted substrate. The Km for each substrate was calculated using concentrations of 0.0025 to 10 mM, and the Km NAD(P)H and NAD(P) was calculated using concentrations of 0.01 to 0.5 mM

*Measurment of enzyme activity:* NADPH-dependent alpha-ketocarbonyl reductases were assayed by a routine method used for NAD(P)-linked enzymes by recording the rate of decrease of NADPH at 340 nm (ε = 6.22 mM⁻¹ x cm⁻¹)) with substrates at 30 °C. The reaction mixture (1 ml) for reduction of alpha-ketocarbonyl substrates contained 10 mM substrate, 40 mM potassium phosphate buffer pH 7.0, 0.025 mM NAD(P)H , and the enzyme. Reactions were induced by addition of enzyme or substrate. One unit of enzyme activity was defined as the amount of enzyme catalyzing the conversion of 1 µmol of pyridine nucleotide per min at 30 °C.

*Cofactor regeneration:* A prerequisite for the production of α-hydroxycarbonyl compounds is the regeneration of the expensive coenzymes and the stability of the protein catalysts. By coupling two coenzyme-dependent enzyme reactions, NADPH can be continuously regenerated. Hence, instead of stoichiometric amounts, only catalytic amounts of coenzyme are required. For this purpose, we used a combined enzymatic reaction between the NADPH-dependent oxidoreductases that form α-hydroxycarbonyl compounds and the aldehyde dehydrogenase GOX1122 (see Schweiger, P. et al., J. Mol. Microbiol. Biotechnol. 13:147-155 (2007)) that produces the reduced cofactor in the course of acetaldehyde oxidation. The aldehyde dehydrogenase has very high turn-over rates and low Kₘ values for both acetaldehyde and NADP and could be produced in high amounts (Schweiger, P. et al., J. Mol. Microbiol. Biotechnol. 13:147-155 (2007)). The biotransformation was performed in a batch process (total volume 2.5 ml 40 mM potassium phosphate buffer pH 7). 30 µg of purified Gox1122 and of NADPH-dependent oxidoreductases and 50 µM NADPH were added. The substrate concentration in the beginning was 20 mM 2,3-pentanedione and 20 mM acetaldehyde. Fresh substrates were added every 60 min (20 mM each).

NMR: The 2.5 ml bioconversion was extracted with two times equal volume of d-chloroform. ¹H and ¹³C NMR spectra were recorded on a DPX 300 Spectrometer (300 MHz) and referenced to the CDCl₃ internal standard. Chemical samples analyzed with Bruker NMR Suite 2.5 software constructed final product structures.

### Example 1: Isolation and preparation of the biocatalysts

The isolation and production of the biocatalysts Gox0313, Gox0502, Gox0644, Gox0646 Gox1615, Gox2107 and Gox2684, which are capable of selectively reducing α-ketocarbonyl compounds into α-hydroxycarbonyl compounds is described in detail in the following. The isolation of Gox1122 for the co-factor regeneration is disclosed in Schweiger, P. et al., J. Mol. Microbiol. Biotechnol. 13:147-155 (2007).
1.1. PCR amplification of genes *gox*0313, *gox*0502, *gox*0644, *gox*0646, *gox*1615 *gox*2107 *and gox*2684: Oligonucleotides containing extended 5' *Bsa*I, *Bbs*I or *BsmB* I restriction sites that correspond to gox0313, gox0502, gox0644, gox0646, gox1615 gox2107 and gox2486 of *G. oxydans* were constructed by utilizing the primers of Table 1. The amplification reaction mixtures contained 100 ng of total DNA of *G. oxydans*, 0.5 pmol of each oligonucleotide primer, 200 µM each deoxynucleotide triphosphate (dNTP), 5 µl of 10X *Pfu* cloned polymerase buffer, and 2.5 U of *Pfu* (Stratagene, La Jolla, CA, USA) in a total volume of 50 µl. The mixture was subjected to PCR amplification in a DNA thermal cycler (Bio-Rad MyCycler) for 30 cycles. Each cycle was defined by a 45-second denaturing step at 95 °C, a 1-minute annealing step at 58 °C, and a 2-minute 30-second polymerization step at 72 °C. PCR products were analyzed by electrophoretic separation in a 0.8 % agarose gel. PCR fragments were microdialyzed with 0.025 µm membrane filters for 1 h.
1.2. Cloning of genes: Vector pASK5-IBA5 contains a N-terminal eight amino acid Strep-tag II that is precisely fused to the structural gene. PCR products and plasmid pASK-IBA5 were subjected to *BsaI* or other appropriate restriction digestion (New England Biolabs, Ipswich, MA, USA). Ligation of the digested vector and PCR fragment was performed with T4 ligase (New England Biolabs, Ipswich, MA, USA), transformed into *E. coli* DH5α cells and plated on LBₐₘₚ selection plates. Plasmids displaying proper digestion patterns were sequenced to confirm correct gene insertion of *gox*0313, *gox*0502, *gox*0644, *gox*0646, *gox*1615, *gox*2107, and *gox*2684 respectively.
1.3. Overexpression and Purification of oxidoreductases: Whole seed cultures of *E. coli* BL21 or *E. coli* DH5α containing plasmid constructs of interest were used to inoculate 1 L MI medium. Cultures were grown at 37 °C and induced by addition of 200 ng/ml anhydrotetracycline at an OD₆₀₀ = 0.4, and allowed to grow for an additional four hours. Cells were harvested by centrifugation at 4500 x g at 4 °C. Cell pellets were resuspended in 15 ml Buffer W (100 mM Tris/HCl pH 8.0, 150 mM NaCl) at 4 °C. Protease cocktail set III (10 µl/L cells) (Merck KGaA, Darmstadt, Germany) and DNase I (20 U/I cells) (Abgene, Epsom, UK) were added to the resuspended cell pellets and allowed to incubate on ice for ten minutes. Resuspended cells were lysed by sonication at 4 °C . Lysates were centrifuged at 24,000 x g at 4 °C and the cleared lysate was collected. Column chromatographies were carried out at 4 °C with a Strep-Tactin Superflow^{®} affinity column (IBA GmbH, Goettingen, Germany). A bed volume of 10 ml was equilibrated with two column volumes of buffer W. Cleared lysates were centrifuged at 12,000 x g at 4 °C for 10 minutes prior to application to the column. The column was washed with five column volumes of Buffer W after the cell extract entered the column. To elute the bound protein Buffer E (Buffer W containing 2.5 mM desthiobiotin) was applied to the column. Elution of the enzymes occurred between 20 -30 ml of Buffer E application.

NADPH-dependent α-ketocarbonyl reductases were assayed by a routine method used for NAD(P)-linked enzymes by recording the rate of decrease of NADPH at 340 nm (ε = 6.22 mM^{-1.}cm⁻¹) with substrates at 30 °C. The reaction mixture (1 ml) for reduction of alpha-ketocarbonyl substrates contained 10 mM substrate, 40 mM potassium phosphate buffer pH 7.0, 0.025 mM NADPH , and the enzyme. Reactions were induced by addition of enzyme or substrate. One unit of enzyme activity was defined as the amount of enzyme catalyzing the conversion of 1 µmol of pyridine nucleotide per min at 30 °C.

Native enzymes Gox0644 and Gox1615 showed single bands when applied to a native PAGE electrophoresis. The peaks corresponded to 66.1 kDa for Gox0644 and 74.5 kDa for Gox1615 (Fig. 2A). These data suggest that both Gox0644 and Gox1615 from *G. oxydans* 621H are active as homodimers. In contrast, native Gox0313 revealed a molecular mass of 181.6 kDa indicating that the proteins exists as a homopentamer.

Gox2107 and Gox0502 showed apparent native molecular weights of 65.2 kDa and 78.2 kDa, respectively, on Native PAGE analysis (Fig. 2B). These data suggests that Gox2107 is active as a homotrimer, whereas Gox0502 is active as a homodimer, which indicates both proteins are folded correctly and are in their native state.

Native enzymes Gox2684 and Gox0646 showed single bands when applied to a native PAGE electrophoresis. The peaks corresponded to 78.4 kDa for Gox2684 and 110 kDa for Gox0646 (Figure 3c). These data suggest that Gox2684 and Gox0646 from G. oxydans 621H are active as homodimers and tetramers, respectively because the masses of the single subunits are 27.7 kDa (Gox0646) and 39.4 kDa (Gox2684).

The proteins were also analysed by SDS-PAGE in order to verify the expected molecular masses of the single subunits by comparison to molecular weight markers. The results are shown in Fig.4 (14 kDa, 21 kDa, 30 kDa, 45 kDa, 55 kDa, 66 kDa, 80 kDa, 97 kDa, 116 kDa and 205 kDa molecular weight markers lane 1). Gox 1122 (lane 2) = 50.5 kDa, Gox 2107 (lane 3) = 21.9 kDa, Gox0502 (lane 4) = 39.2 kDa, Gox1615 (lane 5) = 37.2 kDa, Gox0313 (lane 6) = 36.5 kDa, Gox0644 (lane 7) = 31.7 kDa, Gox0646 (lane 8) = 27.7 and Gox2684 (lane 8) = 39.4 kDa These results were then compared to the expected sizes derived from the known amino acid sequence.

**Table 2: Final Molecular Weights and Subunit Composition**

| Protein | Subunit Mass | Determined Native Mass¹ | Subunit Composition |
|---|---|---|---|
| Gox0313 | 36.5 | 181.6 | Pentamer |
| Gox0502 | 39.2 | 78.2 | Dimer |
| Gox0644 | 31.7 | 66.1 | Dimer |
| Gox0646 | 27.7 | 110 | Tetramer |
| Gox1615 | 37.2 | 74.5 | Dimer |
| Gox2107 | 21.9 | 65.2 | Trimer |
| Gox2684 | 39.4 | 78.4 | Dimer |
| Gox1122 | 50.5 | 50.2 | Monomer |

| | | | |
|---|---|---|---|
| 1) final subunit composition of each protein in their native state. | | | |

The SDS PAGE results verified the purity of the protein sample through the visualization of a single pure band on the gel. The biocatalysts were all correctly aligned with the expected molecular weight markers (Tab. 2).

### Example 2: Characterization of the biocatalysts

For each enzyme (Gox0313, Gox0502, Gox0644, Gox0646, Gox1615, Gox2107, and Gox2684 respectively), fresh enzyme preparations were used to validate the substrate spectrum and the kinetic activity. Each protein exhibited activity with α-ketoaldehydes and/or α-diketones (Tab. 3) a special class of organic compounds capable of chiral formation through regioselective and stereospecific reduction. Purified NAD(P)-dependent oxidoreductases were assayed at 30 °C by a routine method used for NAD(P)-linked enzymes by recording the rate of decrease of NAD(P)H at 340 nm (ε = 6.22 mM/µmol·cm) with substrates capable of reduction reactions. The standard reaction mixture (1 ml) for the reduction reactions of substrates contained 10 mM substrate, 40 mM potassium phosphate buffer (K-phosphate buffer) pH 7.0, 0.05 mM NAD(P)H, and the enzyme unless otherwise noted. Reactions were induced by addition of enzyme or substrate. One unit of enzyme activity was defined as the amount of enzyme catalyzing the conversion of 1 µmol of pyridine nucleotide per min at 30 °C. Measurement of the protein content was performed by the method of Bradford in Anal. Biochem. 72:248-254 (1976).

**Table 3: Summary of enzymatic activities with α-diketones, α-ketoaldehydes and /or α-ketoesters**

| Substrate/Gox | 0313* | 0502* | 0644* | 0646* | 1615* | 2107* | 2684* |
|---|---|---|---|---|---|---|---|
| α-diketones | | | | | | | |
| 2,3-pentanedione | 35.9 | | 89.3 | 8.0 | 6.2 | | 0.6 |
| 2,3-hexanedione | 19.6 | | 50.1 | 8.0 | 6.5 | | 2.3 |
| 3,4-hexanedione | 19.5 | 2.0 | 7.3 | 2.0 | 2.0 | | 0.6 |
| 1-phenyl-1,2-propanedione | 0.2 | 2.0 | 75.4 | 6.0 | 5.4 | | |

| α-keto-aldehydes | | | | | | | |
|---|---|---|---|---|---|---|---|
| phenylglyoxal | 0.5 | 15.7 | 66.0 | | 19.1 | 35.2 | 2.1 |
| methylglyoxal | 10.6 | | 3.4 | 0.2 | 19.3 | | 1.4 |
| α-ketoester | | | | | | | |
| ethylpyruvate | 0.2 | | 92.8 | | 1.2 | | |

| | | | | | | | |
|---|---|---|---|---|---|---|---|
| *Activity in U/mg = U/mg protein = µmol x min⁻¹ x mg protein⁻¹ | | | | | | | |

2.1. Enzymatic activity of Gox0644: Gox0644 utilized NADPH in the reduction reactions of α-diketones, α-ketoaldehydes and α-ketoesters. The α-diketones, 2,3 butanedione, 2,3 pentanedione, 2,3 hexanedione and 1 phenyl-1,2 propanedione displayed the highest activity (Tab. 4). The α-ketoaldehydes phenylglyoxal and methylglyoxal and the keto ester ethyl pyruvate also exhibited high activity. Particular substrates not reduced by Gox0644 were acetoin, 2-oxo butyric acid, acetylacetone, acetone, and simple aldehydes. The results show that Gox0644 carbonyl reduction is unable to achieve activity with β diketones and β ketoacids from fatty acid degradation or with 2-keto acids associated with amino acid catabolism. Furthermore, the results with acetoin reduction or oxidation did not demonstrate activity. This result shows that the α-diketones reaction selectively reduced only one carbonyl group and was irreversible.

**Tab. 4: Enzymatic activity of Gox0644**

| Substrates | Specific Activity (U/mg)* | Cofactor |
|---|---|---|
| glyoxal | 1.1 | NADPH |
| ketogulonic acid | 2.3 | NADPH |
| glyoxalate | 1.2 | NADPH |
| methylglyoxal | 3.4 | NADPH |
| 2,3-butanedione | 60.0 | NADPH |
| 2,3-pentanedione | 89.3 | NADPH |
| 2,3-hexanedione | 50.1 | NADPH |
| phenylglyoxal | 66.0 | NADPH |
| 3,4-hexanedione | 7.3 | NADPH |
| Ethylpyruvate | 92.8 | NADPH |
| 1-phenyl-1,2-propanedione | 75.4 | NADPH |
| Isatin | 16.1 | NADPH |
| benzil | 14.0 | NADPH |
| Pyruvate | 1.63 | NADPH |

| | | |
|---|---|---|
| *U/mg = µmol/mg min | | |

2.2. Enzymatic activity of Gox1615: Gox1615 revealed activity with wide variety of chemical substrates. As can be seen from Tab. 5, Gox1615 specifically used NADPH and displayed the highest activity with the ketoaldehydes phenylglyoxal and methylglyoxal, but also utilized diketones and aldehydes.

**Tab. 5: Enzymatic activity of Biocatalyst Gox1615**

| Substrates | Specific Activity (U/mg)* | Cofactor |
|---|---|---|
| 2-oxobutyrate | 6.5 | NADPH |
| Glyoxal | 5 | NADPH |
| methylglyoxal | 19.3 | NADPH |
| phenylglyoxal | 19.1 | NADPH |
| 3,4-hexanedione | 2 | NADPH |
| 2,3-pentanedione | 6.2 | NADPH |
| ethylpyruvate | 1.2 | NADPH |
| isoamylpyruvate | 4.7 | NADPH |
| 2,3-hexanedione | 6.5 | NADPH |
| 1-phenyl-1,2-propanedione | 5.4 | NADPH |
| 2,3-butanedione | 6 | NADPH |
| 3-penten-2-one | 0.5 | NADPH |
| 3-buten-2-one | 0.7 | NADPH |
| Isatin | 2.4 | NADPH |
| propionaldehyde | 8.4 | NADPH |
| butyraldehyde | 14 | NADPH |
| valeraldehyde | 15.1 | NADPH |
| hexanal | 10.3 | NADPH |
| heptanal | 15.5 | NADPH |
| octanal | 5.3 | NADPH |
| Nonal | 9.3 | NADPH |
| decanal | 10.5 | NADPH |
| undecylaldehyde | 7.5 | NADPH |
| dodecanal | 15.6 | NADPH |
| benzaldehyde | 12.2 | NADPH |
| glutaraldehyde | 13.5 | NADPH |
| glycolaldehyde | 4.1 | NADPH |
| DL-glyceraldehyde | 19 | NADPH |
| isovaleraldehyde | 12 | NADPH |
| p-toluylaldehyde | 2.3 | NADPH |
| m-toluylaldehyde | 1.5 | NADPH |
| o-toluylaldehyde | 0.8 | NADPH |
| phenylacetaldehyde | 6.5 | NADPH |
| 2-methylvaleraldehyde | 13.4 | NADPH |
| 5-methyl-3-hexen-2-one | 0.3 | NADPH |

| | | |
|---|---|---|
| *U/mg = µmol/mg min | | |

2.3. Enzymatic activity of Gox0313: Gox0313 utilized a wide variety of chemical substrates in reduction and oxidation reactions with the cofactor NADH/NAD+ (Tab. 6). Furthermore, the enzyme also uses NADPH as reductant, however with slower reaction rates. Gox0313 reduced many aldehydes, α diketones, and α ketoaldehydes. Gox0313 was also oxidized a number of primary alcohols and diols including ethanol and 1,2 propanediol as well as sugars like glucose and mannose

**Tab. 6: Enzymatic activity of Biocatalyst Gox0313**

| Substrate Reduction | Specific Activity (U/mg)* | Cofactor |
|---|---|---|
| 2,3-butanedione | 28.5 | NADH |
| 2,3-pentanedione | 35.9 | NADH |
| 2,3-hexanedione | 19.6 | NADH |
| 3,4-hexanedione | 19.5 | NADH |
| methylglyoxal | 10.6 | NADH |
| phenylglyoxal | 0.5 | NADH |
| 1-phenyl-1,2-popanedione | 0.2 | NADH |
| ethylpyruvate | 0.1 | NADH |
| isoamylpyruvate | 0.1 | NADH |
| Benzil | 0.1 | NADH |
| acetoin | 10.5 | NADH |
| 3-penten-2-one | 1.5 | NADH |
| 3-buten-2-one | 1.9 | NADH |
| propionaldehyde | 15.0 | NADH |
| glucose | 4.7 | NADH |
| mannose | 9.7 | NADH |
| acetaldehyde | 61.0 | NADH |
| butyraldehyde | 8.5 | NADH |
| valeraldehyde | 8.9 | NADH |
| hexanal | 6.0 | NADH |
| heptanal | 8.6 | NADH |
| octanal | 1.5 | NADH |
| Nonal | 4.4 | NADH |
| decanal | 18.8 | NADH |
| undecylaldehyde | 8.5 | NADH |
| dodecanal | 2.4 | NADH |
| cinnamaldehyde | 7.0 | NADH |
| hydrocinnamaldehyde | 32.1 | NADH |
| p-toluoylaldehyde | 3.3 | NADH |
| DL-glyceraldehyde | 1.2 | NADH |
| benzaldehyde | 9.4 | NADH |
| glycolaldehyde | 37.5 | NADH |

| | | |
|---|---|---|
| *U/mg = µmol/mg min | | |

2.4 Enzymatic activity of Gox0502 and Gox2107: The enzymatic activity of Gox0502 and Gox2107 is summarized in Tab. 7.

**Tab. 7: Enzymatic activity of Biocatalyst Gox0502 and Gox2107**

| | Gox0502 (Cofactor NADPH)/Specific Activity (U/mg)* | Gox2107 (Cofactor NADPH)/ Specific Activity (U/mg)* |
|---|---|---|
| 1,4-naphthoquinone | 16.6 | 217.5 |
| p-benzoquinone | 10 | 180.5 |
| 2-methyl-1,4-naphthoquinone | 10 | 124.1 |
| DDQ | < 0.1 | 26.5 |
| 2-hydroxy-1,4-naphthoquinone | 0.75 | 16.04 |
| 3-buten-2-one | 21 | 120 |
| Phenylglyoxal | 15.7 | 35.2 |
| 2-cyclohexene-1-one | 9.8 | 3.3 |
| (+)-Carvone | 8.0 | < 0.1 |
| 1-pentene-3-one | 6.1 | 14.0 |
| 3-pentene-2-one | 3.6 | 1.9 |
| 1-phenyl-1,2-propanedione | 2,0 | < 0.1 |
| 3,4-hexanedione | 2,0 | < 0.1 |
| Methylglyoxal | 1.5 | < 0.1 |
| 2,3-hexanedione | 1.0 | < 0.1 |
| 2,3-butanedione | 0.5 | < 0.1 |

| | | |
|---|---|---|
| *U/mg = µmol/mg min | | |

Enzymatic assays indicate that both enzymes are able to reduce vinyl ketones, quinones, and α-ketoaldehydes in the presence of nicotinamide-adenine dinucleotides. The substrate spectrum of the proteins was examined for an in depth analysis of enzymatic activities. Gox2107 showed substrate specificity towards α-ketoaldehydes (phenylglyoxal), short chain vinyl ketones and unsubstituted quinones with a preference for NADPH (table 7), displaying 7.5-fold decrease in activity with NADH (data not shown). Generally, the reaction rate decreased as the number of carbons preceding the reactive vinyl-keto group increased. For quinones, the reaction rate generally decreased as the number substituted carbons on the ring increased. Accordingly, Gox2107 optimal reduced 3-butene-2-one, the shortest possible vinyl ketone, and 1,4-napthoquinone with activities of 119 µmol/mg min and 217.4 µmol/mg min, respectively (table 7). High activities were also obtained with phenylglyoxal (a α-ketoaldehyde). Gox0502 and Gox2684 exhibited similar substrate specificity to Gox2107, however Gox0502 and Gox2684 displayed a strict preference for NADPH (table 7). The preferred substrates were 3-butene-2-one, phenylglyoxal and 1,4-napthoquinone. Gox0502 and Gox2684 enzymes were also able to reduce α-diketones in a selective manner. The production of butane-2-one from 3-buten-2-one was confirmed with NMR for both proteins, confirming the reduction of vinyl-ketones to aliphatic ketones
2.5 Enzymatic activity of GOX2684: The enzymatic activity of GOX2684 is summarized in Tab. 7 and is essentially the same as Gox0502.

**Tab. 8: Enzymatic activity of GOX2684**

| Substrates | Specific Activities (U/mg)* | Cofactor |
|---|---|---|
| Methylglyoxal | 1.4 | NADPH |
| Phenylqlyoxal | 2.1 | NADPH |
| 2,3-butanedione | 0.5 | NADPH |
| 2,3-pentanedione | 0.6 | NADPH |
| 2,3-hexandione | 2.3 | NADPH |
| 3,4-hexanedione | 0.6 | NADPH |
| 3-penten-2-one | 1.9 | NADPH |
| 1,4-napthaloquinone | 3.4 | NADPH |
| 2-hydroxy-1,4-napthoquinone | 0.7 | NADPH |
| 2-methyl-1,4-napthoquinone | 6.8 | NADPH |
| hydrocinnamaldehyde | 1.9 | NADPH |
| Cinnamaldehyde | 0.4 | NADPH |
| 3-buten-2-one | 8.8 | NADPH |

2.6 Enzymatic activity of GOX0646: The enzymatic activity of GOX0646 is summarized in Tab. 9.

**Tab. 9: Enzymatic activity of GOX0646**

| Substrates | Specific Activities (U/mg)* | Cofactor |
|---|---|---|
| L2,3-hexandione | 8.0 | NADPH |
| 2,3-pentanedione | 8.0 | NADPH |
| 2,3-butanedione | 0.8 | NADPH |
| 3,4-hexanedione | 2.0 | NADPH |
| 1-phenyl-1,2-propanedione | 6.0 | NADPH |
| Isatin | 0.1 | NADPH |
| methylglyoxal | 0.2 | NADPH |
| 1,4-napthaloquinone | 0.2 | NADPH |
| p-benzoquinone | 0.3 | NADPH |

2.7 Regioselectivity of GOX0644 and GOX1615: Tab. 10 displays the regioselective reduction product of Gox0644 and Gox1615 as determined by NMR analysis. Both specifically reduced the 2-ketone group of the diketone to form the 2-hydroxy-3-ketone product. Gox0644 also displayed the ability to selectively reduce the 2-ketone group of phenylglyoxal and preserved the aldehyde group in the formation of 2-hydroxy-2-phenylacetaldehyde.

**Tab. 10: Summary of Regioselectivity**

| Enzyme | Substrate | Product |
|---|---|---|
| Gox0644 | 2,3-pentanedione | 2-hydroxy-pentane-3-one |
| Gox0644 | 2,3-hexanedione | 2-hydroxy-hexane-3-one |
| Gox1615 | Phenylglyoxal | 2-hydroxy-2-phenylacetaldehyde |
| Gox1615 | 2,3-pentanedione | 2-hdrox-pentane-3-one |

2.8 Kinetic parameters of the keto carbonyl reductases

The Michaelis constants were determined from purified enzyme by Lineweaver-Burk plots. The Kₘ and Vmax values for the substrates were calculated using concentrations of 0.025 to 10 mM, The optimal pH values and temperatures for enzyme activities are also shown in Tab. 11.

**Tab. 11: Summary of kinetic parameters**

| Protein | Substrate | Vmax (µmol/min•mg) | Km (mM) | Temp. (°C) | pH |
|---|---|---|---|---|---|
| Gox0313 | 2,3 pentanedione | 97.0 | 31 | 30 | 7.0 |
| Gox0502 | 3-butene 2-one | 21.0 | 0.1 | 30 | 7.0 |
| Gox0644 | 2,3 pentanedione | 79.0 | 5.6 | 37 | 6.8 |
| | Phenylglyoxal | 93.0 | 4.7 | 37 | 6.8 |
| Gox0646 | 2,3 pentanedione | 10.0 | 9.3 | 32 | 7.0 |
| Gox1615 | 2,3 hexanedione | 6.0 | 3.2 | 30 | 6.5 |
| | Methylglyoxal | 22.0 | 3.8 | 30 | 6.5 |
| Gox2107 | 3-butene 2-one | 119 | 3.8 | 35 | 6.0 |
| Gox2684 | 3-butene 2-one | 11.3 | 0.3 | 30 | 7.0 |
| | | | | | |
| Gox1122 | Acetaldehyde | 196 | 0.2 | 70 | 8.0 |

### Example 3: Description of the Cofactor Regeneration Systems

3.1. Isolation: The *Gluconobacter oxydans* 621H genome contains a gene (gox1122) that encodes a putative cytosolic NAD(P)-dependent aldehyde dehydrogenase (isolation and characterization (see Schweigert, P. et al, J. Mol. Microbiol.&Biotech.203:1-9 (2007)). The gene was expressed in *Escherichia coli*, and the recombinant enzyme was purified and characterized. The native protein Gox1122 exhibited an apparent mol. mass of 50.1 kDa, and the subunit mass was 50.5 kDa, indicating a monomeric structure of the native enzyme. The preferred substrates were acetaldehyde and NADP forming acetate and NADPH. The enzyme also oxidized other short-chained aliphatic and aromatic aldehydes at lower rates
3.2 Development of the cofactor regeneration system: A prerequisite for the production of α-hydroxycarbonyl compounds is the regeneration of the expensive coenzymes and the stability of the protein catalysts. By coupling two coenzyme-dependent enzyme reactions, NADPH can be continuously regenerated. Hence, instead of stoichiometric amounts, only catalytic amounts of coenzyme are required. For this purpose, a combined enzymatic reaction was used between the NADPH-dependent oxidoreductases that form α-hydroxy carbonyl compounds and the aldehyde dehydrogenase GOX1122 that produces the reduced cofactor in the course of acetaldehyde oxidation. The aldehyde dehydrogenase has very high turn-over rates and low Kₘ values for both acetaldehyde and NADP and could be produced in high amounts (Schweiger, P. et al., J. Mol. Microbiol. Biotechnol. 13:147-155 (2007)).
The products derived from the α-diketone and α-ketoaldehyde substrate reaction were generated according to the cofactor regeneration protocol. This method overcomes enzymes dependence on reduced cofactor by coupling the substrate reactions of Gox0502, Gox0644, Gox0646, Gox1615, Gox2107 and Gox2684 with cofactor NADP reduction reactions based on acetaldehyde oxidoreductase Gox1122 using acetaldehyde. The results showed that Gox0644 remained very active even at high concentrations (50 mM) of acetaldehyde (Tab. 12). The acetaldehyde tolerance tests showed that at high concentrations (100 mM) the activity of Gox0644 did not show a dramatic decrease. In both the reactions with phenylglyoxal and 2,3 pentanedione, Gox0644 activity remained similar to the control reaction consisting of a reaction without acetaldehyde (Tab. 12/13). Gox0644 with phenylglyoxal and 100 mM of acetaldehyde did not show more than a 20% decrease in activity. In the reaction with 2,3 pentanedione there was a slight decrease of about 20% from the control, however, the activity levels were still very high at 66.8 U/mg. This test showed the ability of Gox0644 to withstand high concentrations of acetaldehyde and allows the use of Gox0644 in a cofactor regeneration reaction in the presence of acetaldehyde.

**Table 12: Gox0644 acetaldehyde tolerance tests with phenylglyoxal**

| Gox0644 with phenylglyoxal | |
|---|---|
| Concentration of acetaldehyde | Gox0644 activity* |
| Control | 38.5 U/mg |
| 10mM | 29.6 U/mg |
| 30mM | 37.1 U/mg |
| 50mM | 39.3 U/mg |
| 100mM | 31.2 U/mg |
| | U=µmol/min |

| | |
|---|---|
| * Gox0644 with phenylglyoxal demonstrates high activity in the presence of high concentrations of acetaldehyde. | |

**Table 13: Gox0644 acetaldehyde tolerance test with 2,3 pentanedione**

| Gox0644 with 2,3-pentanedione | |
|---|---|
| Concentration of acetaldehyde | Gox0644 activity* |
| Control | 87.6 U/mg |
| 10mM | 90.5 U/mg |
| 30mM | 79.3 U/mg |
| 50mM | 66.8 U/mg |
| | U=µmol/mg.min |

| | |
|---|---|
| *Gox0644 with 2,3 pentanedione demonstrates high activity in the presence of high concentrations of acetaldehyde. This assay displays the enzyme's high tolerance to acetaldehyde without a decrease in activity with the desired substrate. | |

3.3. Cofactor regeneration systems:
Biotransformations were carried out with Gox1122 oxidizing acetaldehyde to acetate with the concomitant reduction of NADP. This reaction was coupled to NADPH-dependent reductases (e.g. Gox0644 and Gox1615) that reduce carbonyls to recycle the cofactor and allow the addition of cofactor in catalytic amounts instead of stoichometric amounts. Biotransformation reactions were sampled at various time points and checked for remaining substrate by standard NADP-linked enzyme assays (Schweiger, P. et al., J. Mol. Microbiol. Biotechnol. 13:147-155 (2007)). The amount of remaining substrate was calculated by assuming a 1:1 stoichometry of cofactor oxidation/reduction to the corresponding substrate oxidation/reduction.

The bioconversion reaction was followed for 3 hours to produce quantities between 10-50 mg of product based on the reaction rate.
The cofactor regeneration system for protein Gox644 utilized the oxidative reaction of protein Gox1122 converting acetaldehyde to acetic acid and reducing NADP+ to NADPH. The reduced NADPH was oxidized in the reduction of diketones to the hydroxy ketone by protein Gox644.

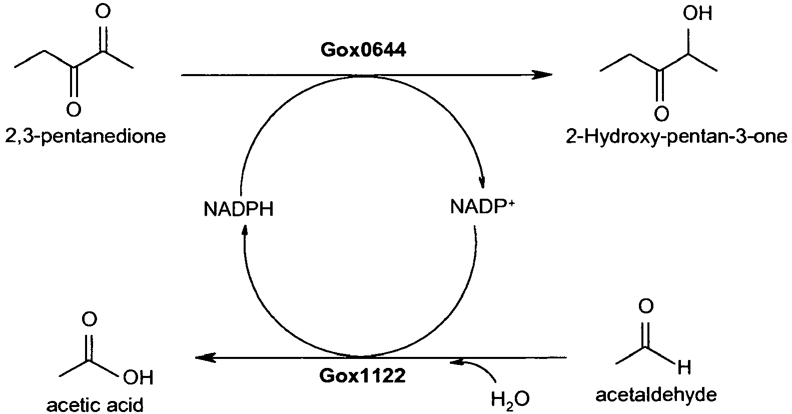

5 ml bioconversions for determining regioselectivity were set up with 2 mg of proteins Gox0644 and Gox1122, 40 mM potassium phosphate buffer, pH 7.0, 50 µM NADPH. Substrates (20 mM 2,3 pentanedione and acetaldehyde) were added every 30 min. The reaction was followed for 3 h to produce quantities between 40-60 mg of product (hydroxy-pentanone). NMR product determination showed that Gox0644 specifically reduced the α-ketone group of 2,3 pentanedione to the corresponding 2-hydroxy-pentan-3-one.

The cofactor regeneration system for protein Gox1615 utilized for the oxidative reaction of protein Gox1122 converting acetaldehyde to acetic acid and reducing NADP+ to NADPH. The reduced NADPH was oxidized in the reduction of phenylglyoxal to 2-hydroxy-2-phenyl-acetaldehyde by protein Gox1615. 5 ml bioconversions for determining regioselectivity were set up with 2 mg of proteins Gox1615 and Gox1122, 40 mM potassium phosphate buffer, pH 7.0, 50 µM NADPH. Substrates (20 mM phenylglyoxal and acetaldehyde) were added every 30 min. The reaction was followed for 3 h to produce quantities between 40-60 mg of product (2-phenyl-2-hydroxyaldehyde). NMR product determination showed that Gox1615 preserved the aldehyde group in the formation of 2-hydroxy-2-phenyl-acetaldehyde.

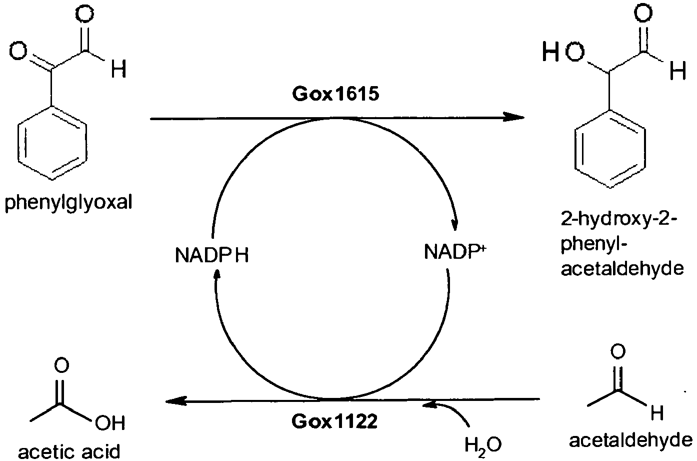

### Sequence Listing - Free Text

| SEQ ID NO: | Description |
|---|---|
| 1/2 | *G. oxydans* α-ketocarbonyl reductase Gox0313 DNA and protein |
| 3/4 | *G. oxydans* α-ketocarbonyl reductase Gox0502 DNA and protein |
| 5/6 | *G. oxydans* α-ketocarbonyl reductase Gox0644 DNA and protein |
| 7/8 | *G. oxydans* α-ketocarbonyl reductase Gox0646 DNA and protein |
| 9/10 | *G. oxydans* α-ketocarbonyl reductase Gox1615 DNA and protein |
| 11/12 | *G. oxydans* α-ketocarbonyl reductase Gox2107 DNA and protein |
| 13/14 | G. oxydans α-ketocarbonyl reductase Gox2684 DNA and protein |
| 15/16 | *G. oxydans* aldehyde dehydrogenase Gox1122 DNA and protein |
| 17-32 | primer |
| 33 | pASK-IBA5 expression vector having: |
| | promoter bp 37-72, |
| | forward primer binding site bp 57-76, |
| | Strep-tag bp 160-192, |
| | multiple cloning site bp 193-274, |
| | reverse primer binding site bp342-358, |
| | f1 origin bp 371-809, |
| | AmpR resistance gene bp 958-1818 and |
| | Tet-repressor 1828-2451). |

## Claims

1. A process for the enzymatic regio- and enantioselective production of α-hydroxy carbonyl compounds which comprises reacting an α-ketocarbonyl compound with an α-ketocarbonyl reductase derived from *Gluconobacter oxydans*, wherein the α-ketocarbonyl reductase
(i) has an apparent molecular weight of 20 to 52 kDa and an activity to selectively reduce the α-keto moiety in α-ketoaldehydes and/or α-ketoesters and/or the keto moiety of a vicinal diketone that carries the shorter alkyl chain of the molecule; and
(ii) comprises the amino acid sequence of Gox0313 (SEQ ID NO:2), Gox0502 (SEQ ID NO:4), Gox0644 (SEQ ID NO:6), Gox0646 (SEQ ID NO:8) Gox1615 (SEQ ID NO:10), Gox2107 (SEQ ID NO:12), Gox2684 (SEQ ID NO:14), or a mutant thereof having at least 80% sequence identity with the native reductases of SEQ ID NO:2, 4, 6, 8, 10, 12 or 14 and having the enzymatic activity of the respective native reductase of SEQ ID NO:2, 4, 6, 8, 10, 12 or 14.

2. The process of claim 1, wherein the reaction is conducted in a cell free environment, wherein the α-ketocarbonyl reductase is present as free enzyme or is presented as a cell lysate or as immobilized enzyme, or the reaction is conducted in the presence of transformants that produce the α-ketocarbonyl reductase *in-situ*.

3. The process of claim 1 or 2, wherein the α-ketocarbonyl compound is selected from α-diketones, α-keto acids, α-keto esters, α-ketoaldehydes and vinylogous/phenylogous compounds thereof.

4. The process of any one of claims 1 to 3, wherein the reaction is conducted in the presence of an reducing cofactor, in equimolar or catalytic amount.

5. The process of claim 4, wherein the reducing cofactor is NADH or NADPH.

6. The process of any one of claims 1 to 5, wherein the amount of α-ketocarbonyl reductase in the reaction mixture relative to the α-ketocarbonyl compound is in the range of 1 mM to 1 M.

7. The process of any one of claims 1 to 6, wherein the reaction is performed in the batch mode or the continuous mode.

8. The process according to any one of claims 1 to 7, wherein
(i) the α-ketocarbonyl reductase is Gox0313 (SEQ ID NO:2) or a mutant thereof having at least 80% sequence identity with the native reductases of SEQ ID NO:2 and having the enzymatic activity of the native reductase of SEQ ID NO:2, and the ketocarbonyl compound to be reduced is an α-diketoneor an α-ketoaldehyde; or
(ii) the α-ketocarbonyl reductase is Gox0502 (SEQ ID NO:4) or a mutant thereof having at least 80% sequence identity with the native reductases of SEQ ID NO:4 and having the enzymatic activity of the native reductase of SEQ ID NO:4, and the ketocarbonyl compound to be reduced is an α-diketone or an α-ketoaldehyde; or
(iii) the α-ketocarbonyl reductase is Gox0644 (SEQ ID NO:6) or a mutant thereof having at least 80% sequence identity with the native reductases of SEQ ID NO:6 and having the enzymatic activity of the native reductase of SEQ ID NO:6, and the ketocarbonyl compound to be reduced is an α-diketone, an α-keto ester or an α-ketoaldehyde; or
(iv) the α-ketocarbonyl reductase is Gox0646 (SEQ ID NO:8) or a mutant thereof having at least 80% sequence identity with the native reductases of SEQ ID NO:8 and having the enzymatic activity of the native reductase of SEQ ID NO:8, and the ketocarbonyl compound to be reduced is an α-diketone or an α-ketoaldehyde; or
(v) the α-ketocarbonyl reductase is Gox1615 (SEQ ID NO:10) or a mutant thereof having at least 80% sequence identity with the native reductases of SEQ ID NO:10 and having the enzymatic activity of the native reductase of SEQ ID NO:10, and the ketocarbonyl compound to be reduced is an α-diketone, an α-keto ester or an α-ketoaldehyde ; or
(vi) the α-ketocarbonyl reductase is Gox2107 (SEQ ID NO:12) or a mutant thereof having at least 80% sequence identity with the native reductases of SEQ ID NO:12 and having the enzymatic activity of the native reductase of SEQ ID NO:12, and the ketocarbonyl compound to be reduced is an α-ketoaldehyde; or
(vii) the α-ketocarbonyl reductase is Gox2684 (SEQ ID NO:14) or a mutant thereof having at least 80% sequence identity with the native reductases of SEQ ID NO:14 and having the enzymatic activity of the native reductase of SEQ ID NO:14, and the ketocarbonyl compound to be reduced is an α-diketone, an α-ketoaldehyde or a quinone.

9. The process according to any one of claims 1 to 8, wherein the reducing cofactor NADPH is applied in catalytic amounts and is regenerated with an aldehyde, and an aldehyde dehydrogenase.

10. The process of claim 9, wherein the aldehyde is acetaldehyde.

11. The process of claim 9 or 10, wherein the aldehyde dehydrogenase is an aldehyde dehydrogenase derived from *Gluconobacter oxydans.*

12. The process of claim 11, wherein the aldehyde dehydrogenase derived from *Gluconobacter oxydans* is Gox1122 (SEQ ID NO:16), or a fragment thereof having the activity of the aldehyde dehydrogenase Gox1122, or a mutant thereof having at least 90% sequence identity with the native aldehyde dehydrogenase of SEQ ID NO:16 and having the activity of the aldehyde dehydrogenase Gox1122.

## Patentansprüche

1. Verfahren zur enzymatischen regio- und enantioselektiven Produktion von α-Hydroxycarbonylverbindungen, umfassend das Umsetzen einer α-Ketocarbonylverbindung mit einer α-Ketocarbonyl-Reductase, die von *Gluconobacter oxydans* stammt, wobei die α-Ketocarbonyl-Reductase
(i) ein scheinbares Molekulargewicht von 20 bis 52 kDa und eine Aktivität, die α-Keto-Struktureinheit in α-Ketoaldehyden und/oder α-Ketoestern und/oder diejenige Keto-Struktureinheit eines vicinalen Diketons, die die kürzere Alkylkette des Moleküls trägt, selektiv zu reduzieren, aufweist; und
(ii) die Aminosäuresequenz von Gox0313 (SEQ ID Nr. 2), Gox0502 (SEQ ID Nr. 4), Gox0644 (SEQ ID Nr. 6), Gox0646 (SEQ ID Nr. 8), Gox1615 (SEQ ID Nr. 10), Gox2107 (SEQ ID Nr. 12), Gox2684 (SEQ ID Nr. 14) oder einer Mutante davon, die wenigstens 80% Sequenzidentität mit den nativen Reductasen von SEQ ID Nr. 2, 4, 6, 8, 10, 12 oder 14 aufweist und die enzymatische Aktivität der jeweiligen nativen Reductase von SEQ ID Nr. 2, 4, 6, 8, 10, 12 oder 14 aufweist, umfasst.

2. Verfahren gemäß Anspruch 1, wobei die Reaktion in einer zellfreien Umgebung durchgeführt wird, wobei die α-Ketocarbonyl-Reductase als freies Enzym oder als Zelllysat oder als immobilisiertes Enzym vorliegt, oder die Reaktion in Gegenwart von Transformanten durchgeführt wird, die die α-Ketocarbonyl-Reductase in situ produzieren.

3. Verfahren gemäß Anspruch 1 oder 2, wobei die α-Ketocarbonylverbindung aus α-Diketonen, α-Ketosäuren, α-Ketoestern, α-Ketoaldehyden und vinylogen/phenylogen Verbindungen davon ausgewählt ist.

4. Verfahren gemäß einem der Ansprüche 1 bis 3, wobei die Reaktion in Gegenwart eines reduzierenden Cofaktors in äquimolarer oder katalytischer Menge durchgeführt wird.

5. Verfahren gemäß Anspruch 4, wobei es sich bei dem reduzierenden Cofaktor um NADH oder NADPH handelt.

6. Verfahren gemäß einem der Ansprüche 1 bis 5, wobei die Menge der α-Ketocarbonyl-Reductase in dem Reaktionsgemisch relativ zu der α-Ketocarbonylverbindung im Bereich von 1 mM bis 1 M liegt.

7. Verfahren gemäß einem der Ansprüche 1 bis 6, wobei die Reaktion im diskontinuierlichen Modus oder kontinuierlichen Modus durchgeführt wird.

8. Verfahren gemäß einem der Ansprüche 1 bis 7, wobei
(i) es sich bei der α-Ketocarbonyl-Reductase um Gox0313 (SEQ ID Nr. 2) oder eine Mutante davon, die wenigstens 80% Sequenzidentität mit den nativen Reductasen von SEQ ID Nr. 2 aufweist und die enzymatische Aktivität der nativen Reductase von SEQ ID Nr. 2 aufweist, handelt und die zu reduzierende Ketocarbonylverbindung ein α-Diketon oder ein α-Ketoaldehyd ist; oder
(ii) es sich bei der α-Ketocarbonyl-Reductase um Gox0502 (SEQ ID Nr. 4) oder eine Mutante davon, die wenigstens 80% Sequenzidentität mit den nativen Reductasen von SEQ ID Nr. 4 aufweist und die enzymatische Aktivität der nativen Reductase von SEQ ID Nr. 4 aufweist, handelt und die zu reduzierende Ketocarbonylverbindung ein α-Diketon oder ein α-Ketoaldehyd ist; oder
(iii) es sich bei der α-Ketocarbonyl-Reductase um Gox0644 (SEQ ID Nr. 6) oder eine Mutante davon, die wenigstens 80% Sequenzidentität mit den nativen Reductasen von SEQ ID Nr. 6 aufweist und die enzymatische Aktivität der nativen Reductase von SEQ ID Nr. 6 aufweist, handelt und die zu reduzierende Ketocarbonylverbindung ein α-Diketon, ein α-Ketoester oder ein α-Ketoaldehyd ist; oder
(iv) es sich bei der α-Ketocarbonyl-Reductase um Gox0646 (SEQ ID Nr. 8) oder eine Mutante davon, die wenigstens 80% Sequenzidentität mit den nativen Reductasen von SEQ ID Nr. 8 aufweist und die enzymatische Aktivität der nativen Reductase von SEQ ID Nr. 8 aufweist, handelt und die zu reduzierende Ketocarbonylverbindung ein α-Diketon oder ein α-Ketoaldehyd ist; oder
(v) es sich bei der α-Ketocarbonyl-Reductase um Gox1615 (SEQ ID Nr. 10) oder eine Mutante davon, die wenigstens 80% Sequenzidentität mit den nativen Reductasen von SEQ ID Nr. 10 aufweist und die enzymatische Aktivität der nativen Reductase von SEQ ID Nr. 10 aufweist, handelt und die zu reduzierende Ketocarbonylverbindung ein α-Diketon, ein α-Ketoester oder ein α-Ketoaldehyd ist; oder
(vi) es sich bei der α-Ketocarbonyl-Reductase um Gox2107 (SEQ ID Nr. 12) oder eine Mutante davon, die wenigstens 80% Sequenzidentität mit den nativen Reductasen von SEQ ID Nr. 12 aufweist und die enzymatische Aktivität der nativen Reductase von SEQ ID Nr. 12 aufweist, handelt und die zu reduzierende Ketocarbonylverbindung ein α-Ketoaldehyd ist; oder
(vii) es sich bei der α-Ketocarbonyl-Reductase um Gox2684 (SEQ ID Nr. 14) oder eine Mutante davon, die wenigstens 80% Sequenzidentität mit den nativen Reductasen von SEQ ID Nr. 14 aufweist und die enzymatische Aktivität der nativen Reductase von SEQ ID Nr. 14 aufweist, handelt und die zu reduzierende Ketocarbonylverbindung ein α-Diketon, ein α-Ketoaldehyd oder ein Chinon ist.

9. Verfahren gemäß einem der Ansprüche 1 bis 8, wobei der reduzierende Cofaktor NADPH in katalytischen Mengen angewendet und mit einem Aldehyd und einer Aldehyd-Dehydrogenase regeneriert wird.

10. Verfahren gemäß Anspruch 9, wobei es sich bei dem Aldehyd um Acetaldehyd handelt.

11. Verfahren gemäß Anspruch 9 oder 10, wobei die Aldehyd-Dehydrogenase eine von *Gluconobacter oxydans* stammende Aldehyd-Dehydrogenase ist.

12. Verfahren gemäß Anspruch 11, wobei es sich bei der von *Gluconobacter oxydans* stammenden Aldehyd-Dehydrogenase um Gox1122 (SEQ ID Nr. 16) oder ein Fragment davon, das die Aktivität der Aldehyd-Dehydrogenase Gox1122 aufweist, oder eine Mutante davon, die wenigstens 90% Sequenzidentität mit der nativen Aldehyd-Dehydrogenase von SEQ ID Nr. 16 aufweist und die Aktivität der Aldehyd-Dehydrogenase Gox1122 aufweist, handelt.

## Revendications

1. Procédé pour la production régiosélective et énantiosélective par voie enzymatique de composés α-hydroxycarbonylés qui comprend la réaction d'un composé α-cétocarbonylé avec une α-cétocarbonyle réductase dérivée de *Gluconobacter oxydans,* dans lequel l'α-cétocarbonyle réductase
(i) a un poids moléculaire apparent de 20 à 52 kDa et une activité pour réduire sélectivement la fraction α-cétonique dans des α-cétoaldéhydes et/ou des α-cétoesters et/ou la fraction cétonique d'une dicétone vicinale qui porte la chaîne alkyle plus courte de la molécule ; et
(ii) comprend la séquence d'acides aminés de Gox0313 (SEQ ID N° : 2), Gox0502 (SEQ ID N° : 4), Gox0644 (SEQ ID N° : 6), Gox0646 (SEQ ID N° : 8), Gox1615 (SEQ ID N° : 10), Gox2107 (SEQ ID N° : 12), Gox2684 (SEQ ID N° : 14), ou un mutant de celles-ci ayant au moins 80 % d'identité de séquence avec les réductases natives de SEQ ID N° : 2, 4, 6, 8, 10, 12 ou 14 et ayant l'activité enzymatique de la réductase native respective de SEQ ID N° : 2, 4, 6, 8, 10, 12 ou 14.

2. Procédé selon la revendication 1, dans lequel la réaction est menée dans un environnement acellulaire, dans lequel l'α-cétocarbonyle réductase est présente sous forme d'enzyme libre ou est présente sous forme de lysat cellulaire ou sous forme d'enzyme immobilisée, ou la réaction est menée en présence de transformants qui produisent l'α-cétocarbonyle réductase in *situ*.

3. Procédé selon la revendication 1 ou 2, dans lequel le composé α-cétocarbonylé est sélectionné parmi les α-dicétones, les α-cétoacides, les α-cétoesters, les α-cétoaldéhydes et les composés vinylogues/phénylogues de ceux-ci.

4. Procédé selon l'une quelconque des revendications 1 à 3, dans lequel la réaction est menée en présence d'un cofacteur de réduction, en quantité équimolaire ou catalytique.

5. Procédé selon la revendication 4, dans lequel le cofacteur de réduction est NADH ou NADPH.

6. Procédé selon l'une quelconque des revendications 1 à 5, dans lequel la quantité d'α-cétocarbonyle réductase dans le mélange réactionnel par rapport au composé α-cétocarbonylé se situe dans la plage de 1 mM à 1 M.

7. Procédé selon l'une quelconque des revendications 1 à 6, dans lequel la réaction est réalisée en mode discontinu ou en mode continu.

8. Procédé selon l'une quelconque des revendications 1 à 7, dans lequel
(i) l'α-cétocarbonyle réductase est Gox0313 (SEQ ID N° : 2) ou un mutant de celle-ci ayant au moins 80 % d'identité de séquence avec les réductases natives de SEQ ID N° : 2 et ayant l'activité enzymatique de la réductase native de SEQ ID N° : 2, et le composé cétocarbonylé à réduire est une α-dicétone ou un α-cétoaldéhyde ; ou
(ii) l'α-cétocarbonyle réductase est Gox0502 (SEQ ID N° : 4) ou un mutant de celle-ci ayant au moins 80 % d'identité de séquence avec les réductases natives de SEQ ID N° : 4 et ayant l'activité enzymatique de la réductase native de SEQ ID N° : 4, et le composé cétocarbonylé à réduire est une α-dicétone ou un α-cétoaldéhyde ; ou
(iii) l'α-cétocarbonyle réductase est Gox0644 (SEQ ID N° : 6) ou un mutant de celle-ci ayant au moins 80 % d'identité de séquence avec les réductases natives de SEQ ID N° : 6 et ayant l'activité enzymatique de la réductase native de SEQ ID N° : 6, et le composé cétocarbonylé à réduire est une α-dicétone, un α-cétoester ou un α-cétoaldéhyde ; ou
(iv) l'α-cétocarbonyle réductase est Gox0646 (SEQ ID N° : 8) ou un mutant de celle-ci ayant au moins 80 % d'identité de séquence avec les réductases natives de SEQ ID N° : 8 et ayant l'activité enzymatique de la réductase native de SEQ ID N° : 8, et le composé cétocarbonylé à réduire est une α-dicétone ou un α-cétoaldéhyde ; ou
(v) l'α-cétocarbonyle réductase est Gox1615 (SEQ ID N° : 10) ou un mutant de celle-ci ayant au moins 80 % d'identité de séquence avec les réductases natives de SEQ ID N° : 10 et ayant l'activité enzymatique de la réductase native de SEQ ID N° : 10, et le composé cétocarbonylé à réduire est une α-dicétone, un α-cétoester ou un α-cétoaldéhyde ; ou
(vi) l'α-cétocarbonyle réductase est Gox2107 (SEQ ID N° : 12) ou un mutant de celle-ci ayant au moins 80 % d'identité de séquence avec les réductases natives de SEQ ID N° : 12 et ayant l'activité enzymatique de la réductase native de SEQ ID N° : 12, et le composé cétocarbonylé à réduire est un α-cétoaldéhyde ; ou
(vii) l'α-cétocarbonyle réductase est Gox2684 (SEQ ID N° : 14) ou un mutant de celle-ci ayant au moins 80 % d'identité de séquence avec les réductases natives de SEQ ID N° : 14 et ayant l'activité enzymatique de la réductase native de SEQ ID N° : 14, et le composé cétocarbonylé à réduire est une α-dicétone, un α-cétoaldéhyde ou une quinone.

9. Procédé selon l'une quelconque des revendications 1 à 8, dans lequel le cofacteur de réduction NADPH est appliqué en quantités catalytiques et est régénéré avec un aldéhyde, et une aldéhyde déshydrogénase.

10. Procédé selon la revendication 9, dans lequel l'aldéhyde est l'acétaldéhyde.

11. Procédé selon la revendication 9 ou 10, dans lequel l'aldéhyde déshydrogénase est une aldéhyde déshydrogénase dérivée de *Gluconobacter oxydans.*

12. Procédé selon la revendication 11, dans lequel l'aldéhyde déshydrogénase dérivée de *Gluconobacter oxydans* est Gox1122 (SEQ ID N° : 16), ou un fragment de celle-ci ayant l'activité de l'aldéhyde déshydrogénase Gox 1122, ou un mutant de celle-ci ayant au moins 90 % d'identité de séquence avec l'aldéhyde déshydrogénase native de SEQ ID N° : 16 et ayant l'activité de l'aldéhyde déshydrogénase Gox1122.
